# EUROPÄISCHE PATENTSCHRIFT

(11) **EP 0 992 235 B1**
(45) Veröffentlichungstag und Bekanntmachung des Hinweises auf die Patenterteilung: **05.11.2003**
(21) Anmeldenummer: 99116625.7
(22) Anmeldetag: 25.08.1999
(51) Int. Cl.: A61K 7/06

(54) **Kosmetisches Mittel**
Cometic composition
Composition cosmétique

(30) Priorität: 26.08.1998 DE 19838851
(43) Veröffentlichungstag der Anmeldung: 12.04.2000
(73) Patentinhaber: BASF AKTIENGESELLSCHAFT, 67056 Ludwigshafen (DE)
(72) Erfinder: Nguyen Kim, Son, 69502 Hemsbach (DE); Sanner, Axel, 67227 Frankenthal (DE); Hössel, Peter, 67105 Schifferstadt (DE); Dausch, Wilma, 67117 Limburgerhof (DE)
(74) Vertreter: Kinzebach, Werner, Dr.

(56) Entgegenhaltungen:
- FR-A- 2 739 282
- US-A- 4 237 253

## Beschreibung

Die vorliegende Erfindung betrifft ein kosmetisches Mittel, welches wenigstens ein wasserlösliches oder wasserdispergierbares Polymer einpolymerisiert enthält.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarfestigungsmittel werden im Allgemeinen in Form von wässrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, dass sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 15°C) besitzen.

Ein weiterer aktueller Anspruch an Haarbehandlungsmittel ist es, dem Haar ein natürliches Aussehen und Glanz zu verleihen, z.B. auch dann, wenn es sich um von Natur aus besonders kräftiges und/oder dunkles Haar handelt.

Ein Nachteil vieler bekannter Haarfestigerpolymere ist der sogenannte "Flaking"-Effekt, d.h. nach dem Auskämmen bleibt ein weißer, schuppenförmiger Rest auf dem Haar zurück. Dies wird von den Anwendern im Allgemeinen als äußerst unangenehm empfunden. Der "Flaking"-Effekt tritt besonders deutlich bei Personen mit dunkler Haarfarbe und/oder besonders kräftigen Haaren auf. Die Einsatzmöglichkeit von Haarfestigerformulierungen, die diesen Effekt aufweisen, ist somit insbesondere auf dem asiatischen Markt deutlich beeinträchtigt. Als mögliche Ursachen für den "Flaking"-Effekt werden u.a. die chemische Struktur der eingesetzten Haarfestigerpolymere und insbesondere die Partikelgröße des Sprays angesehen. Neben den zuvor genannten Eigenschaften sollen Haarfestigerpolymere daher vorzugsweise eine hohe Treibgasverträglichkeit aufweisen, um eine Formulierung in Spraydosen unter möglichst hohem Druck zuzulassen. Dies gilt sowohl für die klassischen Treibmittel auf Propan/Butan-Basis, als auch für deren Ersatzstoffe, z.B. auf Dimethyletherbasis.

Die EP-A-0 100 890, EP-A-0 257 444, DE-A-40 31 912 und die DE-A-39 01 325 beschreiben Copolymerisate, die wenigstens einen Alkylester der Acrylsäure oder Methacrylsäure, wenigstens ein N-Vinyllactam, z.B. N-Vinylpyrrolidon, sowie weitere Monomere einpolymerisiert enthalten und die Verwendung dieser Copolymerisate in Haarbehandlungsmitteln.

Nachteilig an den zuvor genannten N-Vinylpyrrolidon-haltigen Copolymeren ist, dass N-Vinyllactame, wie N-Vinylpyrrolidon, leicht säurekatalytisch zu nichtpolymerisierbaren Nebenprodukten umgesetzt werden, z.B. mit Ethanol zu 2-Ethoxy-N-ethylpyrrolidon. Diese Nebenprodukte stehen in einem reversiblen Gleichgewicht mit dem freien N-Vinyllactam, so dass im Allgemeinen Produkte mit einem niedrigen Molekulargewicht und einem sehr hohen Restmonomerengehalt erhalten werden.

Die WO 97/00664 beschreibt eine wässrige Nagelpolitur, die ein mit einem difunktionellen Urethanacrylat vernetztes Acrylharz enthält. Die getrockneten Filme sind weder wasserlöslich noch wasserdispergierbar und aufgrund ihrer Propan/Butan-Unverträglichkeit für Haarsprays ungeeignet.

Die EP-A-379 082 beschreibt ein Haarfestigungsmittel, enthaltend als Filmbildner ein Copolymerisat, welches
A) 75 bis 99 Gew.-% tert.-Butyl(meth)acrylat,
B) 1 bis 25 Gew.-% (Meth)acrylsäure und
C) 0 bis 10 Gew.-% eines weiteren radikalisch copolymerisierbaren hydrophoben Monomeren
einpolymerisiert enthält. Haarfestigungsmittel auf Basis dieser Copolymere, die nur die Komponenten A) und B) enthalten machen das Haar zu hart und weisen eine zu geringe Propan/Butan-Verträglichkeit auf. Copolymere, die zusätzlich ein Monomer C) enthalten sind bezüglich ihrer Auswaschbarkeit verbesserungswürdig.

Die DE-A-43 14 305 beschreibt wie die EP-A-379 082 ein Haarfestigerpolymer auf Basis von tert.-Butyl(meth)acrylat und (Meth)acrylsäure, welches 0 bis 60 Gew.-% eines C₁-C₁₈-Alkyl(meth)acrylats oder einer Mischung davon mit N-C₁-C₁₈-Alkyl(meth)acrylamiden einpolymerisiert enthält. Zusätzliche Monomere mit einer Kohlenstoffzahl von mehr als 8 führen zwar unter Umständen zu einer besseren Propan/Butan-Verträglichkeit, wobei jedoch gleichzeitig die Auswaschbarkeit deutlich verschlechtert wird.

Die WO-A 98/00096 hat einen der EP-A-379 082 und DE-A-4 314 305 5 vergleichbaren Offenbarungsgehalt.

Die EP-A-0 372 546 und die EP-A-0 728 778 beschreiben Filmbildnerharze, welche wenigstens ein (Meth)acrylamid, wenigstens ein C₁-C₄-Alkyl(meth)acrylat, wenigstens ein N,N-Dialkyl(meth)acrylat oder N,N-Dialkyl(meth)acrylamid und gegebenenfalls wenigstens ein Hydroxyalkyl(meth)acrylat oder Polyalkylenglykol(meth)acrylat einpolymerisiert enthalten. Copolymere, die eine α,β-ethylenisch ungesättigte Mono- und/oder Dicarbonsäure einpolymerisiert enthalten, sind nicht beschrieben. Diese Copolymere weisen nur eine geringe Löslichkeit in Ethanol auf und die resultierenden Filme sind hart, sodaß bei ihrer Verwendung in Haarfestigern dem Haar kein natürliches Aussehen verliehen wird. Auch ihre Propan/Butan-Verträglichkeit ist verbesserungswürdig.

Die JP-A-57 050 912 beschreibt ein Haarbehandlungsmittel auf Basis eines nichtionischen hydrophilen Oligoalkylenoxid(meth)acrylates mit bis zu 10 Alkylenoxideinheiten, welches das Haar weich macht.

Die JP-A-03 206 023 beschreibt ein Polymerharz für Haarbehandlungsmittel, welches
a) 6 bis 35 Gew.-% Acrylsäure, Methacrylsäure, Itaconsäure oder eine Mischung davon,
b) 15 bis 50 Gew.-% wenigstens eines C₁₀-C₁₈-Alkyl(meth)acrylats,
c) 15 bis 50 Gew.-% wenigstens eines C₄-C₈-Alkyl(meth)acrylats und
d) 0 bis 25 Gew.-% wenigstens eines weiteren hydrophoben Vinylmonomers
einpolymerisiert enthält. Die erhaltenen Copolymere werden mit einer Base neutralsiert. Wie die in der EP-A-379 082 und DE-A-4 314 305 beschriebenen Haarfestigerpolymere weisen auch diese Copolymere einen hohen Anteil an hydrophoben Monomeren auf. Ihre Auswaschbarkeit ist daher verbesserungswürdig.

Die JP-A-03 206 024 beschreibt ein der JP-A-03 206 023 vergleichbares Haarfestigerpolymer, das zusätzlich 5 bis 50 Gew.-% eines N-Alkyl-substituierten Acrylamids einpolymerisiert enthält. Auch die Auswaschbarkeit dieser Polymere ist verbesserungswürdig.

Die JP-A-01 213 221 beschreibt ein Haarfärbemittel, enthaltend ein Tetrapolymer, das
a) 30 bis 70 Gew.-% wenigstens eines (Meth)acrylsäureesters der allgemeinen Formel 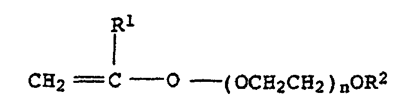 R¹ = H, CH₃; R² = CH₃, C₂H₅; n = 1-10
b) 5 bis 25 Gew.-% Acrylsäure, Methacrylsäure und/oder Itaconsäure,
c) 5 bis 20 Gew.-% wenigstens eines C₈-C₁₈-Alkylesters der Acrylsäure und/oder Methacrylsäure,
d) 20 bis 50 Gew.-% wenigstens eines weiteren Vinylmonomers, ausgewählt unter n-Butyl(meth)acrylat, Isobutyl(meth)acrylat, Cyclohexyl(meth)acrylat, Vinylacetat, Vinylpyrrolidon, Diaceton(meth)acrylamid, Acrylnitril oder Styrol,

einpolymerisiert enthält und welches anschließend mit einer wasserlöslichen organischen Base neutralisiert wird. Polymere, die einen tert.-Butylester oder ein N-tert.-Butylamid einer α,β-ethylenisch ungesättigten Carbonsäure aufweisen, werden nicht beschrieben. Diese Polymere bilden aufgrund des hohen Alkylenoxidanteils zu weiche Filme und eignen sich daher nicht als Haarfestiger. Zudem ist ihre LPG-Verträglichkeit verbesserungswürdig.

Der vorliegenden Erfindung liegt die Aufgabe zugrunde, neue kosmetische Mittel, insbesondere Haarbehandlungsmittel, zur Verfügung zu stellen, die eine hohe Treibgasverträglichkeit aufweisen und im Wesentlichen keinen "Flaking"-Effekt zeigen. Vorzugsweise sollen diese Mittel dem Haar Glätte und Geschmeidigkeit verleihen.

Überraschenderweise wurde nun gefunden, dass diese Aufgabe durch kosmetische Mittel gelöst wird, die wenigstens ein wasserlösliches oder wasserdispergierbares Polymer enthalten, das wenigstens einen tert.-Butylester und/oder ein N-tert.-Butylamid einer α,β-ethylenisch ungesättigten Carbonsäure, wenigstens eine α,β-ethylenisch ungesättigte Mono- und/oder Dicarbonsäure, wenigstens eine α,β-ethylenisch ungesättigte Verbindung mit mindestens 5 Alkylenoxideinheiten und wenigstens eine α,β-ethylenisch ungesättigte Verbindung mit einem C₈-C₃₀-Alkyl- oder -Alkylenrest einpolymerisiert enthält.

Gegenstand der vorliegenden Erfindung ist daher ein kosmetisches Mittel, enthaltend wenigstens ein wasserlösliches oder wasserdispergierbares Polymer, das
a) wenigstens ein α,β-ethylenisch ungesättigtes Monomer der allgemeinen Formel I 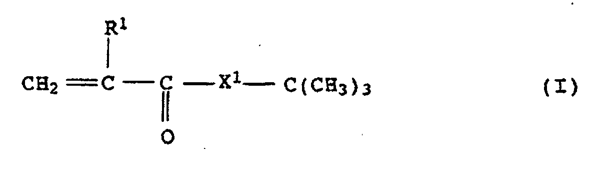 worin
   - R¹: für Wasserstoff oder C₁-C₈-Alkyl steht, und
   - X¹: für O oder NR² steht, wobei R² für Wasserstoff, C₁-C₈-Alkyl oder C₅-C₈-Cycloalkyl steht,
b) wenigstens eine α,β-ethylenisch ungesättigte Mono- und/oder Dicarbonsäure,
c) wenigstens eine Verbindung mit mindestens einer α,β-ethylenisch ungesättigten Doppelbindung und mindestens 5 Alkylenoxideinheiten pro Molekül,
d) wenigstens eine Verbindung mit mindestens einer α,β-ethylenisch ungesättigten Doppelbindung und mindestens einem geradkettigen oder verzweigten C₈-C₃₀-Alkyl- oder -Alkylenrest pro Molekül,
   einpolymerisiert enthält, wobei die Komponenten c) und/oder d) teilweise oder vollständig durch eine Komponente e) ersetzt sein können, wobei
e) für wenigstens eine Verbindung mit mindestens einer α,β-ethylenisch ungesättigten Doppelbindung, mindestens 5 Alkylenoxideinheiten und mindestens einem geradkettigen oder verzweigten C₈-C₃₀-Alkyl- oder -Alkylenrest pro Molekül steht,
oder die Salze davon.

Die in den erfindungsgemäßen kosmetischen Mitteln eingesetzten wasserlöslichen oder wasserdispergierbaren Polymere können zusätzlich bis zu 10 Gew.-%, bezogen auf die Gesamtmenge der zu polymerisierenden Monomere, wenigstens eines weiteren radikalisch copolymerisierbaren Monomers einpolymerisiert enthalten.

Im Rahmen der vorliegenden Erfindung umfasst der Ausdruck C₁-C₈-"Alkyl" geradkettige und verzweigte Alkylgruppen. Vorzugsweise handelt es sich dabei um geradkettige oder verzweigte C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl etc.

C₈-C₃₀-Alkyl bzw. C₈-C₃₀-Alkylen steht vorzugsweise für geradkettige und verzweigte Alkyl- bzw. Alkylengruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc.

Bei der C₅-C₈-Cycloalkylgruppe handelt es sich z.B. um Cyclopentyl, Cyclohexyl, Cycloheptyl oder Cyclooctyl.

### Komponente a)

Bei der Komponente a) handelt es sich bevorzugt um α,β-ethylenisch ungesättigte Verbindungen der allgemeinen Formel I, worin
- R¹: für Wasserstoff, Methyl oder Ethyl steht, und
- X¹: für O oder NR² steht, wobei R² für Wasserstoff, Methyl, Ethyl, n-Propyl, Isopropyl, n-Butyl, sec.-Butyl, tert.-Butyl oder Cyclohexyl steht.

Dabei können auch Mischungen von Verbindungen der Komponente a) eingesetzt werden.

Bevorzugt handelt es sich bei der Komponente a) um tert.-Butylacrylat, tert.-Butylmethacrylat, tert.-Butylethacrylat, N-tert.-Butylacrylamid, N-tert.-Butylmethacrylamid, N-tert.-Butylethacrylamid und Mischungen davon.

### Komponente b)

Geeignete α,β-ethylenisch ungesättigte Mono- und Dicarbonsäuren sind z.B. Acrylsäure, Methacrylsäure, Fumarsäure, Maleinsäure, Itaconsäure, Crotonsäure und Mischungen davon. Bevorzugt werden Acrylsäure, Methacrylsäure und Mischungen davon eingesetzt.

### Komponente c)

Bevorzugt ist die Komponente c) ausgewählt unter
Polyetheracrylaten der allgemeinen Formel II 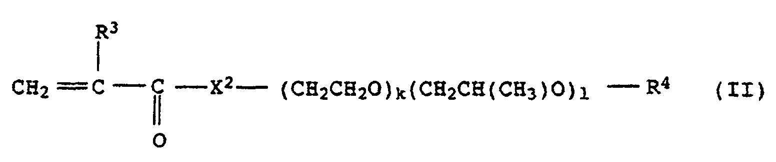 worin
die Reihenfolge der Alkylenoxideinheiten beliebig ist,
- k und l: unabhängig voneinander für eine ganze Zahl von 0 bis 50 stehen, wobei die Summe aus k und l mindestens 5 beträgt,
- R³: für Wasserstoff oder C₁-C₈-Alkyl steht, und
- R⁴: für Wasserstoff oder C₁-C₆-Alkyl steht,
- X²: für O oder NR² steht, wobei R² für Wasserstoff, C₁-C₈-Alkyl oder C₅-C₈-Cycloalkyl steht.

Bevorzugt handelt es sich bei den Polyetheracrylaten c) um Verbindungen der allgemeinen Formel II, worin die Summe aus k und l für eine ganze Zahl von 5 bis 70, bevorzugt 6 bis 50, steht.

In der Formel II steht R³ bevorzugt für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R⁴ in der Formel II für Wasserstoff, Methyl, Ethyl, n-Propyl, Isopropyl, n-Butyl, sec-Butyl, n-Pentyl oder n-Hexyl.

Vorzugsweise steht X² in der Formel II für O oder NH.

Geeignete Polyetheracrylate c) sind z.B. die Polykondensationsprodukte der zuvor genannten α,β-ethylenisch ungesättigten Monound/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R⁴-OH hergestellt werden. Die Alkylenoxide können einzeln, alternierend nacheinander oder als Mischung eingesetzt werden. Die Polyetheracrylate c) können allein oder in Mischungen zur Herstellung der erfindungsgemäß eingesetzten Polymere verwendet werden.

### Komponente d)

Bevorzugt ist die Komponente d) ausgewählt unter Verbindungen der allgemeinen Formel III 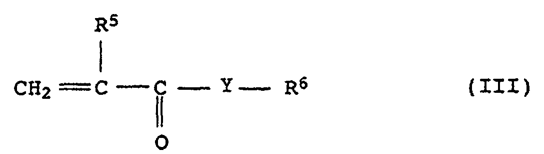 worin
- R⁵: für Wasserstoff oder C₁-C₈-Alkyl steht,
- R⁶: für einen geradkettigen oder verzweigten C₈-C₃₀-Alkylrest steht, und
- Y: für O oder NR⁷ steht, wobei R⁷ für Wasserstoff, C₁-C₈-Alkyl oder C₅-C₈-Cycloalkyl steht.

Vorzugsweise steht in der Formel III R⁵ für Wasserstoff, Methyl oder Ethyl.

Bevorzugt steht Y für O oder NH.

Insbesondere steht R⁶ für n-Octyl, Ethylhexyl, 1,1,3,3-Tetramethylbutyl, Undecyl, Lauryl, Tridecyl, Myristyl, Pentadecyl, Palmityl/ Margarinyl, Stearyl, Palmitoleinyl, Oleyl oder Linolyl.

Insbesondere ist die Komponente d) ausgewählt unter n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth) acrylat, Stearyl(meth)acrylat, Lauryl(meth)acrylat, n-Octyl(meth)acrylamid, 1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid und Mischungen davon.

### Komponente e)

Bevorzugt ist die Komponente e) ausgewählt unter
e1) Polyetheracrylaten der allgemeinen Formel II, wie zuvor bei der Komponente c) definiert, worin R⁴ für C₈-C₃₀-Alkyl steht,
e2) Alkylenoxidgruppen enthaltenden Urethan(meth)acrylaten, und Mischungen davon.

Bevorzugt handelt es sich bei den Polyetheracrylaten e1) um Verbindungen der allgemeinen Formel II, worin die Summe aus k und l für eine ganze Zahl von 5 bis 70, bevorzugt 6 bis 50, steht.

Vorzugsweise hat für die Polyetheracrylate e1) R³ die zuvor bei der Komponente c) genannten bevorzugten Bedeutungen.

Bei den Polyetheracrylaten e1) steht in der Formel II R⁴ vorzugsweise für n-Octyl, Ethylhexyl, 1,1,3,3-Tetramethylbutyl, Undecyl, Lauryl, Tridecyl, Myristyl, Pentadecyl, Palmityl, Margarinyl, Stearyl, Palmitoleinyl, Oleyl oder Linolyl.

Bei den Polyetheracrylaten e1) steht in der Formel II X² vorzugsweise für O oder NH.

Die Herstellung der Polyetheracrylate e1) kann z.B. analog zur Herstellung von c) erfolgen, d.h. durch Umsetzung einer α,β-ethylenisch ungesättigten Mono- und/oder Dicarbonsäure, bzw. eines geeigneten Derivates davon, mit einem Polyetherol, wobei als Startermoleküle zur Herstellung dieser Polyetherole langkettige Alkohole R⁴-OH eingesetzt werden, worin R⁴ für C₈-C₃₀-Alkyl steht. Die Alkylenoxide können wiederum einzeln, alternierend nacheinander oder als Mischung zur Herstellung der Polyetherole eingesetzt werden. Die Polyetheracrylate e1) können allein oder in Mischungen zur Herstellung der erfindungsgemäß eingesetzten Polymere eingesetzt werden.

Bevorzugte Alkylenoxidgruppen enthaltende Urethan(meth)acrylate e2) enthalten die folgenden Verbindungen: f, g und h; oder f, h, i und m; oder g und l; oder i, l und m; oder f, i, l und m; oder f, h, k und m; sowie ggf. weitere Verbindungen eingebaut, wobei
f) für wenigstens ein Diisocyanat steht,
g) für wenigstens eine Verbindung der allgemeinen Formel IV

   R⁸-O-(CH₂CH₂O)ₘ(CH₂CH(CH₃)O)ₙ-H (IV)

   steht, worin
   die Reihenfolge der Alkylenoxideinheiten beliebig ist,
   - R⁸: für einen geradkettigen oder verzweigten C₈-C₃₀-Alkylrest steht,
   - m und n: unabhängig voneinander für eine ganze Zahl von 0 bis 50 stehen, wobei die Summe aus m und n mindestens 5 beträgt,
h) für wenigstens eine α,β-ethylenisch ungesättigte Verbindung steht, die zusätzlich wenigstens eine gegenüber Isocyanatgruppen reaktive Gruppe pro Molekül enthält,
i) für eine Verbindung steht, die ausgewählt ist unter einwertigen Alkoholen, Diolen, Aminen, Diaminen und Aminoalkoholen mit mindestens einem geradkettigen oder verzweigten C₈-C₃₀-Alkyl- oder -Alkylenrest pro Molekül und Mischungen davon,
k) für wenigstens ein aliphatisches, cycloaliphatisches oder aromatisches Monoisocyanat steht,
l) für wenigstens eine α,β-ethylenisch ungesättigte Verbindung, die zusätzlich wenigstens eine Isocyanatgruppe pro Molekül enthält, steht
m) für wenigstens eine Verbindung der allgemeinen Formel V

   R⁹-(CH₂CH₂O)ₚ(CH₂CH(CH₃)O)_{q}-R¹⁰ (V)

   steht, worin
   die Reihenfolge der Alkylenoxideinheiten beliebig ist,
   - p und q: die zuvor für m und n angegebenen Bedeutungen besitzen,
   - R⁹: für OH oder NHR¹¹ steht, wobei R¹¹ für Wasserstoff, C₁-C₈-Alkyl oder C₅-C₈-Cycloalkyl steht,
   - R¹⁰: für H, CH₂CH₂NHR¹¹ oder CH₂CH(CH₃)NHR¹¹ steht.

   Nach einer geeigneten Ausführungsform enthalten die Urethan(meth)acrylate e2) zusätzlich wenigstens eine Komponente eingebaut, die ausgewählt ist unter
n) Verbindungen mit einem Molekulargewicht im Bereich von 56 bis 300, die zwei aktive Wasserstoffatome pro Molekül enthalten,
o) Polytetrahydrofuranen mit zwei aktiven Wasserstoffatomen pro Molekül
p) Polysiloxanen der allgemeinen Formel VI 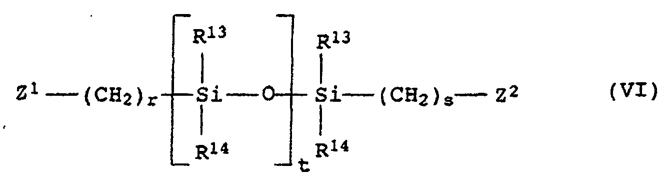 worin
   - R¹³ und R¹⁴: unabhängig voneinander für C₁-C₄-Alkyl, Benzyl, Phenyl oder einen Rest der Formel VII

   -(CH₂)ᵤ-O-(CH₂CH₂O)ᵥ(CH₂CH(CH₃)O)_{w}-H (VII)

   stehen, wobei
   in der Formel VII die Reihenfolge der Alkylenoxideinheiten beliebig ist,
   u für eine ganze Zahl von 1 bis 8 steht,
   v und w unabhängig voneinander für eine ganze Zahl von 0 bis 200 stehen, wobei die Summe aus v und w > 0 ist,
      - Z¹ und Z²: unabhängig voneinander für OH, NHR¹⁵ oder einen Rest der Formel VII stehen, wobei R¹⁵ für Wasserstoff, C₁-C₆-Alkyl oder C₅-C₈-Cycloalkyl steht,
      - r und s: unabhängig voneinander für 2 bis 8 stehen,
      - t: für 3 bis 50 steht,
und Mischungen davon.

Bei der Komponente f) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon. Bevorzugt handelt es sich bei der Komponente f) um Hexamethylendiisocyanat, Isophorondiisocyanat, o- und m-Xylylendiisocyanat, Dicyclohexylmethandiisocyanat und Mischungen davon. Gewünschtenfalls können bis zu 3 Mol-% der genannten Verbindungen durch Triisocyanate ersetzt sein.

Bevorzugt handelt es sich bei der Komponente g) um Verbindungen der allgemeinen Formel IV, worin die Summe aus m und n für eine ganze Zahl von 5 bis 70, bevorzugt 6 bis 50, steht.

Bevorzugt steht R⁸ in der Formel IV für n-Octyl, Ethylhexyl, 1,1,3,3-Tetramethylbutyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, etc.

Geeignete Verbindungen g) sind z.B. die zuvor genannten, zur Herstellung der Komponente e1) geeigneten Polyetherole, wie z.B. Fettalkoholalkoxilate.

Geeignete Monomere h) sind die Ester α,β-ethylenisch ungesättigter Mono- und Dicarbonsäuren, wie Acrylsäure, Methacrylsäure, Fumarsäure, Maleinsäure, Itaconsäure, Crotonsäure etc., mit C₁-C₂₀-Alkandiolen. Dazu zählen z.B. 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc. Vorzugsweise werden Hydroxyethylacrylat und Hydroxyethylmethacrylat eingesetzt. Geeignete Monomere h) sind auch die Ester der zuvor genannten Säuren mit Triolen und Polyolen, wie z.B. Glycerin, Erythrit, Pentaerythrit, Sorbit etc.

Geeignete Monomere h) sind weiterhin die Ester und Amide der zuvor genannten α,β-ethylenisch ungesättigten Mono- und Dicarbonsäuren mit C₂-C₁₂-Aminoalkoholen, die eine primäre oder sekundäre Aminogruppe aufweisen. Dazu zählen Aminoalkylacrylate und Aminoalkylmethacrylate und deren N-Monoalkylderivate, die z.B. einen N-C₁-C₈-Monoalkylrest tragen, wie Aminomethylacrylat, Aminomethylmethacrylat, Aminoethylacrylat, Aminoethylmethacrylat, N-Methylaminomethylacrylat, N-Methylaminomethylmethacrylat, N-Ethylaminomethylacrylat, N-Ethylaminomethylmethacrylat, N-(n-propyl)aminomethyl(meth)acrylat, N-Isopropylaminomethyl-(meth)acrylat und bevorzugt tert.-Butylaminoethylacrylat und tert.-Butylaminoethylmethacrylat. Dazu zählen weiterhin N-(Hydroxy-C₁-C₁₂-alkyl)(meth)acrylamide, wie N-Hydroxymethyl(meth)-acrylamid, N-Hydroxyethl(meth)acrylamid etc.

Geeignete Monomere h) sind auch die Amide der zuvor genannten α,β-ethylenisch ungesättigten Mono- und Dicarbonsäuren mit Di- und Polyaminen, die mindestens zwei primäre oder zwei sekundäre oder eine primäre und eine sekundäre Aminogruppe(n) aufweisen. Dazu zählen z.B. die entsprechenden Amide der Acrylsäure und Methacrylsäure (im Folgenden durch die Silbe "(meth)" bezeichnet), wie Aminomethyl(meth)acrylamid, Aminoethyl(meth)acrylamid, Aminopropyl(meth)acrylamid, Amino-n-butyl(meth)acrylamid, Methylaminoethyl(meth)acrylamid, Ethylaminoethyl(meth)acrylamid, Methylaminopropyl(meth)acrylamid, Ethylaminopropyl(meth)acrylamid, Methylamino-n-butyl(meth)acrylamid etc.

Geeignete einwertige Alkohole i) weisen einen geradkettigen oder verzweigten Alkylrest mit 8 bis 30 Kohlenstoffatomen auf, der gegebenenfalls zusätzlich einfach, zweifach oder mehrfach ungesättigt sein kann. Geeignete C₈-C₃₀-Alkylreste sind die zuvor genannten. Die Alkohole i) können einzeln oder als Gemische eingesetzt werden. Solche Alkohole und Alkoholgemische sind z.B. erhältlich durch Hydrierung von Fettsäuren aus natürlichen Fetten und Ölen oder von synthetischen Fettsäuren, z.B. aus der katalytischen Oxidation von Paraffinen. Geeignete Alkohole und Alkoholgemische i) sind weiterhin erhältlich durch Hydroformylierung von Olefinen mit gleichzeitiger Hydrierung der Aldehyde, wobei im Allgemeinen Gemische aus geradkettigen und verzweigten primären Alkoholen (Oxo-Alkohole) resultieren. Geeignete Alkohole und Alkoholgemische i) sind weiterhin erhältlich durch partielle Oxidation von n-Paraffinen nach bekannten Verfahren, wobei überwiegend lineare sekundäre Alkohole erhalten werden. Geeignet sind weiterhin die durch aluminiumorganische Synthese erhältlichen im Wesentlichen primären, geradkettigen und geradzahligen Ziegler-Alkohole.

Geeignete einwertige Alkohole i) sind z.B. 1-, 2-, 3- und 4-Octanol, 1-, 2-, 3-, 4- und 5-Nonanol, 1-, 2-, 3-, 4- und 5-Decanol, 1-, 2-, 3-, 4-, 5- und 6-Undecanol, 1-, 2-, 3-, 4-, 5-, 6- und 7-Dodecanol, 1-, 2-, 3-, 4-, 5-, 6-, 7- und 8-Tridecanol, 1-, 2-, 3-, 4-, 5-, 6-, 7-, 8- und 9-Tetradecanol, 1-, 2-, 3-, 4-, 5-, 6-, 7-, 8-, 9- und 10-Pentadecanol, 1-, 2-, 3-, 4-, 5-, 6-, 7-, 8-, 9-, 10- und 11-Hexadecanol, 1-, 2-, 3-, 4-, 5-, 6-, 7-, 8-, 9-, 10-, 11- und 12-Heptadecanol, 1-, 2-, 3-, 4-, 5-, 6-, 7-, 8-, 9-, 10-, 11-, 12- und 13-Octadecanol, etc. und Mischungen davon.

Geeignete Diole i) weisen wenigstens einen der zuvor genannten geradkettigen oder verzweigten C₈-C₃₀-Alkylenreste auf. Dazu zählen z.B. 1,2-; 1,3-; 1,4-; 1,5-; 1,6-; 1,7- und 1,8-Octandiol, 1,2- bis 1,9-Nonandiol, 1,2- bis 1,10-Decandiol, 1,2- bis 1,11-Undecandiol, 1,2- bis 1,12-Dodekandiol, 1,2- bis 1,13-Tridecandiol, 1,2- bis 1,14-Tetradecandiol, 1,2- bis 1,15-Pentadecandiol, 1,2- bis 1,16-Hexadecandiol, 1,2- bis 1,17-Heptadecandiol, 1,2- bis 1,18-Octadecandiol, etc. und Mischungen davon.

Geeignete höhere primäre oder sekundäre Amine i) sind Amine und Amingemische, die einen oder zwei der zuvor genannten C₈-C₃₀-Alkylreste aufweisen. Diese können z.B. durch Umsetzung natürlicher oder synthetischer Fettsäuren oder Fettsäuregemische mit Ammoniak zu Nitrilen und anschließende Hydrierung erhalten werden. Dazu zählen z.B. Alkylamine, die die zuvor bei den einwertigen Alkoholen i) genannten Alkylreste aufweisen, das heißt die isomeren Octyl-, Nonyl-, Decyl-, Undecyl-, Dodecyl-, Tridecyl-, Tetradecyl-, Pentadecyl-, Hexadecyl-, Heptadecyl-, Octadecylamine etc. und Mischungen davon.

Geeignete Diamine i) und Aminoalkohole i) können die zuvor bei den Diolen i) genannten Alkylenreste aufweisen. Dabei handelt es sich dann um die isomeren Octan-, Nonan-, Decan-, Undecan-, Dodecan-, Tridecan-, Tetradecan-, Hexadecan-, Heptadeca-, Octadecandiamine und Aminalkohole etc. und Mischungen davon.

Geeignete Monoisocyanate k) sind z.B. C₈-C₃₀-Alkylisocyanate, die aus den zuvor genannten Aminen und Amingemischen durch Phosgenierung oder aus natürlichen oder synthetischen Fettsäuren und Fettsäuregemischen durch Hofmann-, Curtius- oder Lossen-Abbau erhältlich sind.

Geeignete cycloaliphatische Monoisocyanate k) sind z.B. Cyclohexylisocyanat, 2-, 3- und 4-Methylcyclohexylisocyanat, etc. und Mischungen davon.

Geeignete aromatische Monoisocyanate k) sind z.B. Phenylisocyanat, 2-, 3- und 4-Methylphenylisocyanat, etc. und Mischungen davon.

Bei der Komponente l) handelt es sich z.B. um Isocyanate der allgemeinen Formel VIII 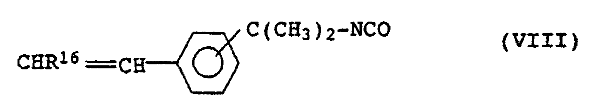 worin
die -C(CH₃)₂-NCO-Gruppe in o-, m- oder p-Stellung zur Vinylgruppe stehen kann und R¹⁶ für Wasserstoff oder C₁-C₈-Alkyl steht.

Bevorzugt steht in der Formel VIII R¹⁶ für Wasserstoff, Methyl oder Ethyl.

Bevorzugt enthalten die Urethan(meth)acrylate e2) als Komponente m) eine Verbindung der allgemeinen Formel V eingebaut, worin R⁹ für OH oder NHR¹¹ steht und R¹⁰ für H, CH₂CH₂NHR¹¹ oder CH₂CH(CH₃)NHR¹¹ steht, wobei R¹¹ für Wasserstoff, Methyl, Ethyl, n-Propyl, n-Butyl, tert.-Butyl oder Cyclohexyl steht.

Die Herstellung dieser Polyether m), die zwei gegenüber Isocyanatgruppen reaktive Gruppen aufweisen, erfolgt z.B. analog zur Herstellung der in den Polyetheracrylaten c) bzw. e1) eingesetzten Polyetherolen. Dazu kann als Startermolekül Wasser oder eine Verbindung mit zwei aktiven Wasserstoffatomen, wie z.B. ein Diol oder primäres oder sekundäres Diamin mit Ethylenoxid und/oder 1,2-Propylenoxid, einzeln, alternierend nacheinander oder als Mischung, umgesetzt werden. Verbindungen der Formel V, die an den Kettenenden wenigstens einen Rest NHR¹¹ aufweisen, sind durch Aminierung von OH-terminierten Polyalkylenoxiden mit Ammoniak oder primären Aminen herstellbar. Geeignete Verbindungen der Formel V sind z.B. die Cremophor®A-Marken und Lutensol®AT-Marken der BASF AG.

Bei der Komponente n) handelt es sich bevorzugt um Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Bevorzugt werden als Komponente n) Diole eingesetzt. Brauchbare Diole sind z.B. Ethylenglykol, Propylenglykol, Butylenglykol, Neopentylglykol, Cyclohexandimethylol, Di-, Tri-, Tetra-, Pentaoder Hexaethylenglykol und Mischungen davon. Bevorzugt werden Neopentylglykol und/oder Cyclohexandimethylol eingesetzt.

Geeignete Aminoalkohole n) sind z.B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine n) sind z.B. Ethylendiamin, Propylendiamin, 1,4-Diaminobutan, 1,5-Diaminopentan und 1,6-Diaminohexan sowie α,ω-Diaminopolyether, die durch Aminierung von Polyalkylenoxiden mit Ammoniak herstellbar sind.

Bei der Komponente o) handelt es sich bevorzugt um ein Polytetrahydrofuran mit einem zahlenmittleren Molekulargewicht im Bereich von etwa 300 bis 5000, bevorzugt etwa 400 bis 4000, insbesondere 500 bis 3000. Geeignete Polytetrahydrofurane o) können durch kationische Polymerisation von Tetrahydrofuran in Gegenwart von sauren Katalysatoren, wie z.B. Schwefelsäure oder Fluoroschwefelsäure, hergestellt werden. Derartige Herstellungsverfahren sind dem Fachmann bekannt.

Nach einer geeigneten Ausführungsform weisen die Polysiloxane p) der allgemeinen Formel VI keine Alkylenoxidreste der allgemeinen Formel VII auf.

Die Polysiloxane p) weisen dann vorzugsweise ein zahlenmittleres Molekulargewicht im Bereich von etwa 300 bis 5000, bevorzugt 400 bis 3000 auf.

Vorzugsweise stehen R¹³ und R¹⁴ dann unabhängig voneinander für C₁-C₄-Alkyl, wie Methyl, Ethyl, n-Propyl, Isopropyl, n-Butyl, sec.-Butyl oder tert.-Butyl. Vorzugsweise stehen R¹³ und R¹⁴ beide für Methyl.

Z¹ und Z² stehen vorzugsweise für OH oder NH₂.

Nach einer weiteren geeigneten Ausführungsform handelt es sich bei den Polysiloxanen p) um Silicon-poly(alkylenoxid)-Copolymere, wobei wenigstens einer oder mehrere der Reste Z¹, Z², R¹³ und/oder R¹⁴ für einen Rest der allgemeinen Formel VII stehen.

Vorzugsweise ist in der Formel VII die Summe aus v und w so gewählt, daß das Molekulargewicht der Polysiloxane p) dann in einem Bereich von etwa 300 bis 30 000 liegt.

Bevorzugt liegt die Gesamtzahl der Alkylenoxideinheiten der Polysiloxane p), das heißt die Summe aus v und w in der Formel VII dann in einem Bereich von etwa 3 bis 200, bevorzugt 5 bis 180.

Bevorzugt sind die übrigen Reste R¹³ und/oder R¹⁴ unabhängig von-einander ausgewählt unter Methyl, Ethyl, n-Propyl, Isopropyl, n-Butyl, sec-Butyl, tert-Butyl, Pentyl, Isopentyl, Hexyl, Octyl, 2-Ethylhexyl, Decyl, Dodecyl und Octadecyl, Cyclohexyl, Phenyl, Naphthyl, Benzyl, Phenylethyl, Tolyl, Xylyl etc.

Bevorzugt steht wenigstens einer der Reste R¹³ oder R¹⁴ für Methyl.

Geeignete Silicon-poly(alkylenoxid)-Copolymere p), die unter dem internationalen Freinamen Dimethicon bekannt sind, sind die Tegopren® -Marken der Fa. Goldschmidt, Belsil® 6031 der Fa. Wacker und Silvet® L der Fa. Witco.

Nach einer bevorzugten Ausfürungsform werden zur Herstellung der in den erfindungsgemäßen Mitteln eingesetzten wasserlöslichen oder wasserdispergierbaren Polymere Alkylenoxidgruppen enthaltende Urethan(meth)acrylate e2) eingesetzt, die
- wenigstens ein Diisocyanat f),
- wenigstens eine Verbindung g), und
- wenigstens eine α,β-ethylenisch ungesättigte Verbindung h), die zusätzlich mindestens ein aktives Wasserstoffatom pro Molekül enthält,
eingebaut enthalten.

Nach einer weiteren bevorzugten Ausführungsform enthalten diese Urethan(meth)acrylate dann zusätzlich wenigstens eine Komponente eingebaut, die ausgewählt ist unter den zuvor genannten Verbindungen n), o) und p) und Mischungen davon.

Die Herstellung der Alkylenoxidgruppen enthaltenden Urethan(meth)acrylate e2) erfolgt durch Umsetzung von Verbindungen, die ausgewählt sind unter den Komponenten g), h), i), m) und Mischungen davon und gegebenenfalls n), o), p) und Mischungen davon, die jeweils wenigstens eine gegenüber Isocyanatgruppen reaktive Gruppe pro Molekül enthalten, mit wenigstens einer isocyanatgruppenhaltigen Verbindung f), k) und/oder l). Die Auswahl der Komponenten erfolgt dabei nach der Maßgabe, daß die Urethan(meth)acrylate e2) mindestens eine α,β-ethylenisch ungesättigte Doppelbindung (Komponenten h und l), mindestens 5 Alkylenoxideinheiten (Komponenten g und m) und mindestens einen geradkettigen oder verzweigten C₈-C₃₀-Alkyl- oder -Alkylenrest (Komponenten g, i und k) pro Molekül sowie mindestens eine Urethan- oder Harnstoffgruppe (abgeleitet von Komponenten f, k und/oder l) enthalten. Bevorzugte urethan(meth)acrylate e2) enthalten die Verbindungen f, g und h; f, h, i und m; g und l; i, l und m; f, i, l und m; f, h, k und m sowie gegebenenfalls weitere Verbindungen die ausgewählt sind unter den Verbindungen der Komponenten f) bis p) und Mischungen davon, eingebaut. Geeignete weitere Kombinationen sind alle, die die zuvor genannte Maßgabe für die Urethan(meth)acrylate e2) erfüllen.

Die Reaktionstemperatur liegt im Allgemeinen in einem Bereich von etwa 60 bis 140°C, bevorzugt etwa 70 bis 100°C. Die Reaktion kann ohne Lösungsmittel oder in einem geeigneten inerten Lösungsmittel oder Lösungsmittelgemisch erfolgen. Geeignete Lösungsmittel sind aprotische polare Lösungsmittel, z.B. Tetrahydrofuran, Essigsäureethylester, n-Methylpyrrolidon, Dimethylformamid und bevorzugt Ketone, wie Aceton und Methylethylketon. Vorzugsweise erfolgt die Reaktion unter einer Inertgasatmosphäre, wie z.B. unter Stickstoff. Die Komponenten werden bevorzugt in solchen Mengen eingesetzt, dass das Verhältnis von NCO-Äquivalent der Verbindungen der Komponenten f), k) und/oder l) zu Äquivalent aktives Wasserstoffatom der Komponenten g), h), i) und/oder m) und, falls vorhanden, n), o) und/oder p) in einem Bereich von etwa 0,8:1 bis 1,25:1, insbesondere 1,05:1 bis 1,15:1, liegt. Weisen die resultierenden Urethan(meth)acrylate c3) noch freie Isocyanatgruppen auf, so werden diese abschließend durch Zusatz von Alkoholen, wie Methanol, Ethanol, etc., Aminen, Aminoalkoholen oder Mischungen davon inaktiviert. Geeignete Aminoalkohole sind die zuvor als n) beschriebenen, bevorzugt 2-Amino-2-methyl-1-propanol.

Bevorzugt weisen die Urethan(meth)acrylate e2) ein zahlenmittleres Molekulargewicht im Bereich von etwa 400 bis 8000, bevorzugt 500 bis 6000, auf.

Bevorzugt weisen die Urethan(meth)acrylate e2) mindestens eine, wie z.B. eine, zwei oder mehrere, α,β-ethylenisch ungesättigte Doppelbindungen pro Molekül auf.

Bevorzugt enthält das in den kosmetischen Mitteln eingesetzte wasserlösliche oder wasserdispergierbare Polymer
- 40 bis 85 Gew.-%, bevorzugt 45 bis 80 Gew.-%, wenigstens einer Komponente a),
- 10 bis 30 Gew.-%, bevorzugt 15 bis 25 Gew.-%, wenigstens einer Komponente b),
- 1 bis 20 Gew.-%, bevorzugt 2 bis 15 Gew.-%, wenigstens einer Komponente c),
- 1 bis 30 Gew.-%, bevorzugt 2 bis 25 Gew.-%, wenigstens einer Komponente d),
einpolymerisiert, wobei die Komponenten c) und/oder d) teilweise oder vollständig durch eine Komponente e) ersetzt sein können.

Die in den erfindungsgemäßen kosmetischen Mitteln eingesetzten wasserlöslichen oder wasserdispergierbaren Polymere können bis zu 10 Gew.-%, bezogen auf die Gesamtmenge der zu polymerisierenden Monomere, wenigstens eines weiteren radikalisch polymerisierbaren Monomers einpolymerisiert enthalten. Dazu zählen z.B. Ester von Vinylalkohol und Allylalkohol mit C₁-C₄₀-Monocarbonsäuren, Vinylether, Vinylaromaten, Vinylhalogenide, Vinylidenhalogenide, C₂-C₈-Monoolefine, nichtaromatische Kohlenwasserstoffe mit mindestens 2 konjugierten Doppelbindungen, N-Vinylamide, N-Vinyllactame, primäre Amide α,β-ethylenisch ungesättigter Monocarbonsäuren, vinyl- und allylsubstituierten heteroaromatischen Verbindungen und Mischungen davon.

Nach einer bevorzugten Ausführungsform handelt es sich um ein Polymer aus:
- 40 bis 85 Gew.-%, bevorzugt 45 bis 80 Gew.-%, wenigstens einer Komponente a),
- 10 bis 30 Gew.-%, bevorzugt 15 bis 28 Gew.-%, wenigstens einer Komponente b),
- 1 bis 20 Gew.-%, bevorzugt 2 bis 15 Gew.-%, wenigstens einer Komponente c),
- 1 bis 30 Gew.-%, bevorzugt 2 bis 25 Gew.-% wenigstens einer Komponente d).

Nach einer weiteren bevorzugten Ausführungsform handelt es sich um ein Polymer aus:
- 40 bis 85 Gew.-%, bevorzugt 45 bis 80 Gew.-%, wenigstens einer Komponente a),
- 10 bis 30 Gew.-%, bevorzugt 15 bis 28 Gew.-%, wenigstens einer Komponente b),
- 1 bis 40 Gew.-%, bevorzugt 5 bis 35 Gew.-%, insbesondere 10 bis 30 Gew.-%, wenigstens einer Komponente e).

Nach einer weiteren bevorzugten Ausführungsform handelt es sich um ein Polymer aus:
- 40 bis 85 Gew.-%, bevorzugt 45 bis 80 Gew.-%, wenigstens einer Komponente a),
- 10 bis 30 Gew.-%, bevorzugt 15 bis 28 Gew.-%, wenigstes einer Komponente b),
- 0,1 bis 20 Gew.-%, bevorzugt 1 bis 15 Gew.-%, wenigstens einer Komponente c), wie zuvor definiert,
- 1 bis 40 Gew.-%, bevorzugt 5 bis 35 Gew.-%, wenigstens einer Komponente e).

Nach einer weiteren bevorzugten Ausführungsform handelt es sich um ein Polymer aus:
- 40 bis 85 Gew.-%, bevorzugt 45 bis 80 Gew.-%, wenigstens einer Komponente a),
- 10 bis 30 Gew.-%, bevorzugt 15 bis 28 Gew.-%, wenigstens einer Komponente b),
- 0,1 bis 30 Gew.-%, bevorzugt 5 bis 35 Gew.-%, wenigstens einer Komponente d),
- 1 bis 40 Gew.-%, bevorzugt 5 bis 35 Gew.-%, wenigstens einer Komponente e).

Die Herstellung der in den erfindungsgemäßen Mitteln eingesetzten Polymere erfolgt durch radikalische Polymerisation nach üblichen, dem Fachmann bekannten Verfahren. Dazu zählt die radikalische Polymerisation in Masse, Emulsion, Suspension und in Lösung, vorzugsweise die Emulsions- und Lösungspolymerisation. Die Mengen an zu polymerisierenden Verbindungen, bezogen auf Lösungs- bzw. Dispergiermittel, werden dabei im Allgemeinen so gewählt, dass etwa 30 bis 80 Gew.-% Lösungen, Emulsionen oder Dispersionen erhalten werden. Die Polymerisationstemperatur beträgt in der Regel 30 bis 120°C, bevorzugt 40 bis 100°C. Das Polymerisationsmedium für die Lösungspolymerisation kann sowohl nur aus einem organischen Lösungsmittel als auch aus Mischungen aus Wasser und mindestens einem wassermischbaren, organischen Lösungsmittel bestehen. Bevorzugte organische Lösungsmittel sind z.B. Alkohole, wie Methanol, Ethanol, n-Propanol, Isopropanol, n-Butanol, Ketone, wie Aceton und Methylethylketon, Tetrahydrofuran etc. Die Lösungspolymerisation kann sowohl als Batchprozess als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzentrationsgefälles unter Aufrechterhaltung der Polymerisation der Polymerisationszone zuführt.

Als Initiatoren für die radikalische Polymerisation werden übliche Peroxo- oder Azoverbindungen eingesetzt. Dazu zählen z.B. Dibenzoylperoxid, tert.-Butylperpivalat, tert.-Butylper-2-ethylhexanoat, Di-tert.-butylperoxid, 2,5-Dimethyl-2,5-di(tert.-butylperoxy)hexan, aliphatische oder cycloaliphatische Azoverbindungen, z. B. 2,2'-Azobis(isobutyronitril), 2,2'-Azobis(2-methylbutyronitril), 2,2'-Azobis(2,4-dimethylvaleronitril), 1,1'-Azobis(1-cyclohexancarbonitril), 2-(Carbamoylazo)isobutyronitril, 4,4'-Azobis(4-cyanovaleriansäure) und deren Alkalimetall- und Ammoniumsalze, z.B. das Natriumsalz, Dimethyl-2,2'-azobisisobutyrat, 2,2'-Azobis[2-(2-imidazolin-2-yl)propan], 2,2'-Azobis(2-amidinopropan) und die Säureadditionssalze der beiden zuletzt genannten verbindungen, z.B. die Dihydrochloride.

Ferner kommen als Initiatoren Wasserstoffperoxid, Hydroperoxide in Kombination mit Reduktionsmitteln und Persalze in Frage. Geeignete Hydroperoxide sind beispielsweise t-Butylhydroperoxid, t-Amylhydroperoxid, Cumolhydroperoxid und Pinanhydroperoxid jeweils in Kombination mit beispielsweise einem Salz der Hydroxymethansulfinsäure, einem Eisen(II)-Salz oder Ascorbinsäure. Geeignete Persalze sind insbesondere Alkalimetallperoxodisulfate.

Die verwendete Initiatormenge, bezogen auf die Monomere, liegt im Allgemeinen in einem Bereich von etwa 0,1 bis 2 Gew.-% bezogen auf das Gesamtgewicht der zu polymerisierenden Monomere.

Die K-Werte der resultierenden Copolymerisate liegen vorzugsweise in einem Bereich von etwa 15 bis 90, bevorzugt 20 bis 70, insbesondere 25 bis 50 (1 gew.-%ige Lösung in Ethanol). Zur Erzielung des gewünschten K-Wertes kann, insbesondere bei der Emulsionsoder Suspensionspolymerisation, der Einsatz eines Reglers angebracht sein. Als Regler eignen sich beispielsweise Aldehyde, wie Formaldehyd, Acetaldehyd, Propionaldehyd, n-Butyraldehyd und Isobutyraldehyd, Ameisensäure, Ammoniumformiat, Hydroxylammoniumsulfat und Hydroxylammoniumphosphat. Weiterhin können Regler eingesetzt werden, die Schwefel in organisch gebundener Form enthalten, wie Di-n-butylsulfid, Di-n-octylsulfid, Diphenylsulfid etc., oder Regler, die Schwefel in Form von SH-Gruppen enthalten, wie n-Butylmercaptan, n-Hexylmercaptan oder n-Dodecylmercaptan. Geeignet sind auch wasserlösliche, schwefelhaltige Polymerisationsregler, wie beispielsweise Hydrogensulfite und Disulfite. Weiterhin eignen sich als Regler Allylverbindungen, wie Allylalkohol oder Allylbromid, Benzylverbindungen, wie Benzylchlorid oder Alkylhalogenide, wie Chloroform oder Tetrachlormethan.

Gewünschtenfalls setzt man der Polymerlösung im Anschluss an die Polymerisationsreaktion einen oder mehrere Polymerisationsinitiatoren zu und erhitzt die Polymerlösung, z.B. auf die Polymerisationstemperatur oder auf Temperaturen oberhalb der Polymerisationstemperatur, um die Polymerisation zu vervollständigen. Geeignet sind die oben angegebenen Azoinitiatoren, aber auch alle anderen üblichen, für eine radikalische Polymerisation in wässriger Lösung geeignete Initiatoren, beispielsweise Peroxide, Hydroperoxide, Peroxodisulfate, Percarbonate, Peroxoester und Wasserstoffperoxid. Hierdurch wird die Polymerisationsreaktion zu einem höheren Umsatz, wie z.B. von 99,9%, geführt. Die bei der Polymerisation entstehenden Lösungen können gegebenenfalls durch ein dem Stand der Technik entsprechendes Trocknungsverfahren in feste Pulver überführt werden. Bevorzugte Verfahren sind beispielsweise die Sprühtrocknung, die Sprühwirbelschichttrocknung, die Walzentrocknung und die Bandtrocknung. Ebenfalls anwendbar sind die Gefriertrocknung und die Gefrierkonzentrierung. Gewünschtenfalls kann das Lösungsmittel auch durch übliche Methoden, z.B. Destillation bei verringertem Druck, teilweise oder vollständig entfernt werden.

Bei den in den erfindungsgemäßen Mitteln eingesetzten wasserlöslichen oder wasserdispergierbaren Polymeren handelt es sich um anionische bzw. anionogene Polymere. Die Säuregruppen der Polymere können mit einer Base teilweise oder vollständig neutralisiert werden. In aller Regel weisen die erhaltenen Salze der Polymere eine bessere Wasserlöslichkeit oder Dispergierbarkeit in Wasser auf als die nicht neutralisierten Polymere. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Geeignete Amine sind z.B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethanolamine, bevorzugt Methyl- oder Ethyldiethanolamin und Di-C₁-C₆-Alkylethanolamine. Besonders für den Einsatz in Haarbehandlungsmitteln haben sich zur Neutralisation der Säuregruppen enthaltenden Polymere 2-Amino-2-methyl-1-propanol, 2-Amino-2-ethylpropan-1,3-diol, Diethylaminopropylamin und Triisopropanolamin bewährt. Die Neutralisation der Säuregruppen enthaltenden Polymere kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 5 bis 95%, vorzugsweise 30 bis 95%, oder vollständig, d.h. zu 100% erfolgen.

Die in den erfindungsgemäßen Mitteln eingesetzten Polymere weisen K-Werte (gemessen nach E. Fikentscher, Cellulose-Chemie 13 (1932), Seite 58-64, an einer 1 gew.-%igen Lösung in Ethanol) in einem Bereich von etwa 15 bis 90, bevorzugt 20 bis 60, auf. Ihre Glasübergangstemperatur beträgt im Allgemeinen mindestens 0°C, bevorzugt mindestens 20°C, insbesondere bevorzugt mindestens 25°C. Üblicherweise liegt die Glasübergangstemperatur dann in einem Bereich von etwa 30 bis 130°C, insbesondere 40 bis 100°C.

Die in den erfindungsgemäßen Mitteln enthaltenen Polymere sind als Hilfsmittel in der Kosmetik und Pharmazie, insbesondere als oder in Beschichtungsmittel(n) für keratinhaltige Oberflächen (Haar, Haut und Nägel) und als Überzugsmittel und/oder Bindemittel für feste Arzneiformen brauchbar. Außerdem sind sie als oder in Beschichtungsmittel(n) für die Textil-, Papier-, Druck-, Leder- und Klebstoffindustrie brauchbar. Sie sind insbesondere in der Haarkosmetik brauchbar. Die zuvor genannten Polymere können auch in Cremes und als Tablettenüberzugmittel und Tablettenbindemittel verwendet werden. Sie eignen sich auch als Bindemittel und Klebemittel für kosmetische Produkte, z.B. bei der Herstellung von Schminken, wie Mascara und Rouge und bei der Herstellung stiftförmiger, kosmetischer Produkte, wie Deostifte, Schminkstifte etc.

Die erfindungsgemäßen kosmetischen Mittel eignen sich insbesondere als Beschichtungsmittel für keratinhaltige Oberflächen (Haar, Haut und Nägel). Die in ihnen eingesetzten Verbindungen sind wasserlöslich oder wasserdispergierbar. Sind die in den erfindungsgemäßen Mitteln eingesetzten Verbindungen wasserdispergierbar, können sie in Form von wässrigen Mikrodispersionen mit Teilchendurchmessern von üblicherweise 1 bis 250 nm, bevorzugt 1 bis 200 nm, zur Anwendung gebracht werden. Die Feststoffgehalte der Präparate liegen dabei üblicherweise in einem Bereich von etwa 0,2 bis 20 Gew.-%, bevorzugt 0,5 bis 12 Gew.-%. Diese Mikrodispersionen benötigen in der Regel keine Emulgatoren oder Tenside zu ihrer Stabilisierung.

Bevorzugt können die erfindungsgemäßen Mittel in Form eines Haarbehandlungsmittels, insbesondere in Form eines Haarsprays vorliegen. Zur Anwendung als Haarfestiger sind dabei Mittel bevorzugt, die Polymere enthalten, die wenigstens eine Glasübergangstemperatur T_{g} ≥ 20°C, bevorzugt ≥ 30°C, aufweisen. Der K-Wert dieser Polymere liegt vorzugsweise in einem Bereich von 23 bis 90, insbesondere 25 bis 60.

Im Allgemeinen enthalten die erfindungsgemäßen Mittel die Polyurethane in einer Menge im Bereich von 0,2 bis 20 Gew.-%, bezogen auf das Gesamtgewicht des Mittels.

Vorzugsweise handelt es sich um Haarbehandlungsmittel. Diese liegen üblicherweise in Form einer wässrigen Dispersion oder in Form einer alkoholischen oder wässrig-alkoholischen Lösung vor. Beispiele geeigneter Alkohole sind Ethanol, Propanol, Isopropanol etc.

Weiter enthalten die erfindungsgemäßen Haarbehandlungsmittel im Allgemeinen übliche kosmetische Hilfsstoffe, beispielsweise Weichmacher, wie Glycerin und Glykol; Emollienzien; Parfüms; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels. Als Treibmittel werden vorzugsweise Kohlenwasserstoffe (LPG), insbesondere Propan, n-Butan, n-Pentan und Gemische davon eingesetzt. Geeignete niedrigsiedende Treibmittel sind weiterhin Ether, bevorzugt Dimethylether. Gewünschtenfalls können als Treibmittel aber auch komprimierte Gase, wie Stickstoff, Luft oder Kohlendioxid, eingesetzt werden. Die in den erfindungsgemäßen Mitteln eingesetzten, zuvor genannten Polymere weisen eine hohe Treibgasverträglichkeit, insbesondere eine hohe Verträglichkeit gegenüber Kohlenwasserstoffen, auf und lassen sich zu Produkten mit einem hohen Treibgasgehalt von z.B. mindestens 40 Gew.-%, bevorzugt mindestens 50 Gew.-%, bezogen auf das Gesamtgewicht des Mittels, formulieren. Im Allgemeinen ist es aber auch möglich, den Treibmittelgehalt gering zu halten, um Produkte mit einem niedrigen VOC-Gehalt zu formulieren. In solchen Produkten beträgt der Treibgasgehalt dann im Allgemeinen nicht mehr als 55 Gew.-%, bezogen auf das Gesamtgewicht des Mittels. Die erfindungsgemäßen Haarfestigungsmittel eignen sich auch für Pumpsprayzubereitungen ohne den Zusatz von Treibmitteln.

Die zuvor beschriebenen Polymere können auch in Kombination mit anderen Haarpolymeren in den Mitteln zur Anwendung kommen. Solche Polymere sind insbesondere:
- nicht-ionische, wasserlösliche bzw. wasserdispergierbare Polymere oder Oligomere, wie Polyvinylcaprolactam, z.B. Luviskol Plus (BASF), oder Polyvinylpyrrolidon und deren Copolymere, insbesondere mit Vinylestern, wie Vinylacetat, z.B. Luviskol VA 37 (BASF); Polyamide, z.B. auf Basis von Itaconsäure und aliphatischen Diaminen;
- amphotere oder zwitterionische Polymere, wie die unter den Bezeichnungen Amphomer® (Delft National) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer, Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), oder
- nichtionische, siloxanhaltige, wasserlösliche oder -dispergierbare Polymere, z.B. Polyethersiloxane, wie Tegopren® (Fa. Goldschmidt) oder Belsil® (Fa. Wacker).

Die erfindungsgemäßen Polymere können mit unvernetzten und vernetzten siloxangruppenhaltigen Polyurethanen und/oder mit wenigstens einem anderen siloxanfreien amidgruppenhaltigen Haarpolymer eingesetzt werden. Dazu zählen z.B. die in der DE-A-42 25 045 beschriebenen Polyurethane, die zuvor beschriebenen Vinylpyrrolidon/Acrylat-Terpolymere und Acrylsäure/Ethylacrylat/N-tert.-Butylacrylamid-Terpolymere (z.B. Ultrahold®strong der BASF AG), die in der DE-A-42 41 118 beschriebenen kationischen Polyurethane, die zuvor beschriebenen amidgruppenhaltigen amphoteren Polymere (z.B. Amphomer® ) und insbesondere Copolymerisate, die einen Anteil an amidgruppenhaltigen Monomeren, wie N-Vinyllactamen, von mindestens 30 Gew.-% aufweisen (z.B. Luviskol® plus und Luviskol® VA37 der BASF AG).

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel enthält:
a) 0,5 bis 20 Gew.-% mindestens eines, in Wasser löslichen oder dispergierbaren Polymers auf Basis von Monomeren der allgemeinen Formel I, wie zuvor beschrieben,
b) 30 bis 99,5 Gew.-%, bevorzugt 40 bis 98 Gew.-%, eines Lösungsmittels, ausgewählt unter Wasser und wassermischbares Lösungsmitteln, bevorzugt C₂-C₅-Alkoholen, insbesondere Ethanol, und Mischungen davon,
c) 0 bis 70 Gew.-% eines Treibmittels, vorzugsweise auf Basis von Propan, n-Butan und/oder n-Pentan bzw. auf Basis von Dimethylether,
d) 0 bis 10 Gew.-% mindestens eines von a) verschiedenen, in Wasser löslichen oder dispergierbaren Haarpolymers,
e) 0 bis 0,3 Gew.-% mindestens eines wasserunlöslichen Silicons,
f) 0 bis 1 Gew.-% mindestens eines nichtionischen, siloxanhaltigen, in Wasser löslichen oder dispergierbaren Polymers,
sowie übliche Zusatzstoffe.

Das erfindungsgemäße Mittel kann als Komponente d) mindestens ein anderes, in Wasser lösliches oder dispergierbares Haarpolymer enthalten. Der Anteil dieser Komponente beträgt dann im Allgemeinen etwa 0,1 bis 15 Gew.-%, bevorzugt 0,1 bis 10 Gew.-%, bezogen auf das Gesamtgewicht des Mittels. Bevorzugt können dabei wasserlösliche oder wasserdispergierbare Polyurethane eingesetzt werden, die gewünschtenfalls zusätzlich Siloxangruppen einpolymerisiert enthalten.

Das erfindungsgemäße Mittel kann als Komponente e) mindestens ein wasserunlösliches Silicon, insbesondere ein Polydimethylsiloxan, z.B. die Abil® -Typen der Fa. Goldschmidt, enthalten. Der Anteil dieser Komponente beträgt dann im Allgemeinen etwa 0,0001 bis 0,2 Gew.-%, bevorzugt 0,001 bis 0,1 Gew.-%, bezogen auf das Gesamtgewicht des Mittels.

Das erfindungsgemäße Mittel kann als Komponente f) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Das erfindungsgemäße Mittel kann zusätzlich gegebenenfalls einen Entschäumer, z.B. auf Silicon-Basis, enthalten. Die Menge des Entschäumers beträgt im Allgemeinen bis zu etwa 0,001 Gew.-%, bezogen auf die Gesamtmenge des Mittels.

Die erfindungsgemäßen Mittel besitzen den Vorteil, dass sie einerseits den Haaren die gewünschte Festigkeit verleihen und andererseits die Polymere leicht auswaschbar (redispergierbar) sind. Dem Haar wird im Allgemeinen ein natürliches Aussehen und Glanz verliehen, auch wenn es sich um von Natur aus besonders kräftiges und/oder dunkles Haar handelt.

Insbesondere lassen sich die erfindungsgemäßen Mittel zu Haarbehandlungsmitteln, insbesondere Haarsprays, mit einem hohen Treibstoffgehalt formulieren. Vorteilhafterweise weisen die erfindungsgemäßen Haarbehandlungsmitteln im Wesentlichen keinen "Flaking"-Effekt auf.

Die Erfindung wird anhand der folgenden, nicht einschränkenden Beispiele näher erläutert.

### Beispiele

### Beispiele 1 bis 3

### Urethan(meth)acrylat Herstellung

In einem Vierhalskolben, der mit Rührer, Tropftrichter, Thermometer, Rückflußkühler und einer Vorrichtung für das Arbeiten unter Stickstoff ausgestattet war, wurde ein ethoxilierter Alkohol in einer Menge nach Tabelle 1 in 100 g Aceton vorgelegt und auf etwa 60°C erhitzt. Anschließend wurde unter Rühren Isophorondiisocyanat in einer Menge nach Tabelle 1 zugetropft und das Reaktionsgemisch dann weitere 60 min unter Rückfluß gerührt. Bei den Beispielen 2 und 3 gab man anschließend, ebenfalls bei etwa 60°C, noch unmittelbar nacheinander Neopentylglycol und Hexamethylendiisocyanat in einer Menge nach Tabelle 1 zu dem Ansatz. Unter Rückfluß wurde das Reaktionsgemisch dann noch so lange gerührt, bis der Isocyanatgruppengehalt des Gemisches praktisch konstant blieb und anschließend unter Rühren auf Raumtemperatur abgekühlt. Gegebenenfalls gab man dann bei einer Temperatur von etwa 30°C ein Polysiloxandiamin (Mₙ = 900 g/mol Tegomer® A-Si 2122 der Fa. Goldschmidt, in Form einer 80%-igen Lösung in Aceton) (Beispiel 3) in einer Menge nach Tabelle 1 zu dem Reaktionsgemisch. In allen Fällen gab man dann tert.-Butylaminoethylmethacrylat in einer Menge nach Tabelle 1 unter Rühren zu dem Reaktionsgemisch, wobei eine Innentemperatur von 40°C nicht überschritten wurde. Nach Zugabe von 300 g Ethanol wurde das Aceton durch Destillation unter verringertem Druck bei etwa 35°C entfernt und dann für die weitere Umsetzung 50 gew.-%ige ethanolische Lösungen der Urethan(meth)acrylate durch Zugabe von weiterem Ethanol hergestellt.

**Tabelle 1**

| Bsp. Nr. | Ethoxilat I¹⁾ [mol] | Ethoxilat II²⁾ [mol] | NPG³⁾ [mol] | Polysiloxandiamin⁴⁾ [mol] | IPDI⁵⁾ [mol] | HDI⁶⁾ [mol] | tert.-BAEMA⁷⁾ [mol] |
|---|---|---|---|---|---|---|---|
| 1 | 1 | - | - | - | 1 | - | 1 |
| 2 | - | 1 | 3 | - | 1 | 3 | 1 |
| 3 | - | 1 | 3 | 1 | 1 | 4 | 1 |

| | | | | | | | |
|---|---|---|---|---|---|---|---|
| 1) C₁₆-, C₁₈-Fettalkoholethoxilat, ca. 11 Ethylenoxideinheiten (Lutensol® AT 11, Fa. BASF AG) | | | | | | | |
| 2) C₁₆-, C₁₈-Fettalkoholethoxilat, ca. 25 Ethylenoxideinheiten (Lutensol® AT 25, Fa. BASF AG) | | | | | | | |
| 3) NPG = Neopentylglycol | | | | | | | |
| 4) Polysiloxandiamin, Mₙ = 900 g/mol (Tegomer® A-Si 2122 der Fa. Goldschmidt) | | | | | | | |
| 5) IPDI = Isophorondiisocyanat | | | | | | | |
| 6) HDI = Hexamethylendiisocyanat | | | | | | | |
| 7) tert.-BAEMA = tert.-Butylaminoethylmethacrylat | | | | | | | |

### Vergleichsbeispiele V4 bis V8, Beispiele 9 bis 20:

### I. Lösungspolymerisation (Beispiele V4-V8, 9-20)

| | | |
|---|---|---|
| Zulauf 1 | 244,8 g | Monomerengemisch nach Tabelle 2 |
| Zulauf 2 | 0,6 g | 2,2'-Azobis(2-methylbutyronitril) |
| | 120 g | Ethanol |
| Zulauf 3 | 2,0 g | 2,2'-Azobis(2-methylbutyronitril) |
| | 120 g | Ethanol |

In einer Rührapparatur mit Rückflußkühler und zwei separaten Zulaufvorrichtungen wurden 48 g Zulauf 1 (Monomerengemisch gemäß Tabelle 2), 16,26 g Zulauf 2 und 120 g Ethanol vorgelegt und die Mischung auf ca. 75°C aufgeheizt. Nach dem Anpolymerisieren, erkennbar an einer beginnenden Viskositätserhöhung, wurde der Rest von Zulauf 1 innerhalb von 3 Stunden und der Rest von Zulauf 2 innerhalb von 4 Stunden zugegeben, wobei die Innentemperatur auf etwa 75 bis 80°C gehalten wurde. Anschließend ließ man 4 Stunden bei 80°C nachreagieren. Danach wurde Zulauf 3 innerhalb von 1/2 Stunde zugegeben, wobei die Innentemperatur auf ca. 80°C erhöht wurde. Nach dem Ende der Zugabe wurde noch 8 Stunden bei dieser Temperatur nachpolymerisiert.

### II. Emulsionspolymerisation (Beispiele V4, V5, 15,17)

Die Polymere der Beispiele V4, V5, 15 und 17 wurden auch durch Emulsionspolymerisation nach folgender Vorschrift hergestellt.

| | | |
|---|---|---|
| Zulauf 1 | 300 g | Monomerengemisch nach Tabelle 2 |
| | 100 g | Wasser |
| | 1 g | Natriumlaurylsulfat |
| | 6 g | Polyethoxisorbitanlaurat (Tween®20, ICI) |
| | 1,2 g | Ethylhexylthioglycolat |
| Zulauf 2 | 0,9 g | Natriumpersulfat |
| | 100 g | Wasser |

In einer Rührapparatur mit Rückflußkühler und zwei separaten Zulaufvorrichtungen wurden 460 g Wasser vorgelegt und auf ca. 75°C aufgeheizt. Dann gab man den voremulgierten Zulauf 1 und den Zulauf 2 innerhalb von 2 Stunden zu, wobei die Temperatur auf etwa 75-80°C gehalten wurde. Gewünschtenfalls kann die Polymerisation durch Zugabe einer wäßrigen Lösung eines Redoxinitiators (tert.-Butylhydroperoxid, Ascorbinsäure) zu dem Reaktionsgemisch und Nachpolymerisieren vervollständigt werden.

**Tabelle 2 :**

| Bsp. Nr. | TBA¹⁾ Gew.-% | MAS²⁾ Gew.-% | AS³⁾ Gew.-% | EA⁴⁾ Gew.-% | PEG-MA⁵⁾ Gew.-% | SMA⁶⁾ Gew.-% | UA-I⁷⁾ Gew.-% | UA-II⁸⁾ Gew.-% | UA-III⁹⁾ Gew.-% | K-Wert¹⁰⁾ |
|---|---|---|---|---|---|---|---|---|---|---|
| V4 | 70 | 23 | - | 7 | - | - | - | - | - | 39,7 |
| V5 | 70 | 23 | - | - | 7 | - | - | - | - | 42,4 |
| V6 | 70 | 23 | - | - | - | 7 | - | - | - | 41,3 |
| V7 | 67 | 23 | - | - | - | 10 | - | - | - | 42,1 |
| V8 | 72 | 21 | - | - | - | 7 | - | - | - | 43,8 |
| 9 | 66 | 22 | - | - | 5 | 7 | - | - | - | 42,2 |
| 10 | 62 | 22 | - | - | 7 | 9 | - | - | - | 45,7 |
| 11 | 62 | 22 | - | - | 7 | 9 | - | - | - | 38,9 |
| 12 | 58 | 23 | - | - | 7 | 10 | - | - | - | 42 |
| 13 | 60 | 23 | - | - | 7 | 12 | - | - | - | 41,2 |
| 14 | 51 | 24 | - | - | 9 | 16 | - | - | - | 42,5 |
| 15 | 60 | 20 | - | - | - | - | 20 | - | - | 40,5 |
| 16 | 57 | 23 | - | - | - | 10 | - | 10 | - | 42,2 |
| 17 | 67 | 23 | - | - | - | - | - | - | 10 | 40,4 |
| 18 | 55 | 25 | - | - | - | 10 | - | - | 10 | 39,8 |
| 19 | 65 | - | 18 | - | 5 | 12 | - | - | - | 38,2 |
| 20 | 58 | - | 20 | - | 7 | 15 | - | - | - | 42,5 |

| | | | | | | | | | | |
|---|---|---|---|---|---|---|---|---|---|---|
| 1) TBA = tert.-Butylacrylat | | | | | | | | | | |
| 2) MAS = Methacrylsäure | | | | | | | | | | |
| 3) AS = Acrylsäure | | | | | | | | | | |
| 4) EA = Ethylacrylat | | | | | | | | | | |
| 5) PEG-MA = Polyethylenglycolmethacrylat (Mg ≈ 350) | | | | | | | | | | |
| 6) SMA = Stearylmethacrylat | | | | | | | | | | |
| 7) - 9) UA-I, -II, -III = Urethan(meth)acrylate der Beispiele 1-3 | | | | | | | | | | |
| 10) 1 gew.-%ige Lösung in Ethanol | | | | | | | | | | |

### Anwendungstechnische Beispiele

### Beispiele V21 bis V23, 24 bis 35

### Aerosol-Haarspray-Formulierungen mit einem VOC-Gehalt von 95 Gew.-%:

| | |
|---|---|
| Polyurethan gemäß Beispielen V6-V8, 9-20 | 5,00 Gew.-% |
| Ethanol | 45,00 Gew.-% |
| Propan/Butan | 49,96 Gew.-% |
| Parfüm, Zusatzstoffe | q.s. |

### Beispiele V36 bis V40, 41 bis 52

### Aerosol-Haarspray-Formulierungen mit einem VOC-Gehalt von 80 Gew.-%:

| Polyurethan gemäß Beispielen | |
|---|---|
| V4-V8, 9-20 | 5,00 Gew.-% |
| Ethanol | 40,00 Gew.-% |
| Wasser | 15,00 Gew.-% |
| Dimethylether | 39,96 Gew.-% |
| Parfüm, Zusatzstoffe | q.s. |

### Beispiele V53 bis V57, 58-69

### Aerosol-Haarspray-Formulierungen mit einem VOC-Gehalt von 55 Gew.-%:

| Polyurethan gemäß Beispielen | |
|---|---|
| V4-V8, 9-20 | 5,00 Gew.-% |
| Ethanol | 20,00 Gew.-% |
| Wasser | 40,00 Gew.-% |
| Propan/Butan | 34,96 Gew.-% |
| Parfüm, Zusatzstoffe | q.s. |

Die Curl-retention der Beispiele V6 bis V8 und 9 bis 20 wurde an der zuvor genannten Haarspray-Formulierung mit einem VOC-Gehalt von 95 gemessen.
- Curl-retention = Festigungswirkung an Haarsträhnen in Lockenform bei hoher Luftfeuchte (90%):

Die Curl-Retention ist ein Maß für die Haarfestigungswirkung. Sie wird im Modellversuch gemessen an Haarlocken, erzeugt durch eine übliche Wasserwelle an ca. 15 cm langen Haaren, die mit Formulierung A) aus 10 cm Entfernung 4 sec lang besprüht werden. Nach 5-stündiger Behandlung der aufgehängten Locken in einer Klimakammer (25°C, 90% relative Luftfeuchte) wird die relative Verformung (Aufweitung) der Locken, bezogen auf die ursprüngliche Form, festgestellt. Ein hoher Wert bedeutet hohe Festigungswirkung, d.h. 100% entspricht einem Erhalt der ursprünglichen Form der aufgehängten Locke, 0% entspricht einem völlig gestreckten Haar. Die Ergebnisse sind in Tabelle 4 wiedergegeben.

An den Polymeren aus den Vergleichsbeispielen V4-V8 sowie den erfindungsgemäßen Beispielen 9-20 wurde die n-Heptanverträglichkeit als Maß für ihre Treibmittelverträglichkeit bestimmt. Dazu wurden je 1,5 g neutralisiertes Polymer und 23,5 g Ethanol zu 6 gew.-%igen Lösungen formuliert und bei Raumtemperatur mit n-Hexan titriert, bis eine Trübung auftritt. Die Ergebnisse sind ebenfalls in Tabelle 4 wiedergegeben.

Die Polymere aus den Vergleichsbeispielen V4-V8 sowie aus den erfindungsgemäßen Beispielen 9-20 wurden zu 5 gew.-%igen ethanolischen Lösungen formuliert. Sie wurden auf eine Glasplatte aufgetragen und die resultierenden Filme wurden im Hinblick auf drei Kriterien, die in Tabelle 3 angegeben sind, geprüft und mit Noten von 1-4 bewertet. Die Bewertungen der Filme sind ebenfalls in Tabelle 4 wiedergegeben.

**Tabelle 3:**

| | | Note |
|---|---|---|
| A) Elastizität | hart und spröde | 4 |
| | hart | 3 |
| | mittel | 2 |
| | elastisch | 1 |
| B) Klebrigkeit | klebrig | 4 |
| | leicht klebrig (weich) | 3 |
| | leicht klebrig (hart) | 2 |
| | nicht klebrig | 1 |
| C) Glätte | bremsend | 4 |
| | mäßig-glatt | 3 |
| | glatt | 2 |
| | sehr glatt | 1 |
| D) Auswaschbarkeit | schlecht | 4 |
| | mäßig | 3 |
| | gut | 2 |
| | sehr gut | 1 |

**Tabelle 4:**

| Polymer aus Bsp. Nr. | Curl-retention [%] | n-Heptan- Verträglichkeit [%] | A | B | C | D |
|---|---|---|---|---|---|---|
| V4 | 84 | 50 | 3-4 | 1 | 3 | 1-2 |
| V5 | 77 | 49 | 3*-4 | 2 | 2-3 | 1 |
| V6 | 87 | 60 | 3 1 | | 2-3 | 3 |
| V7 | 88 | 64 | 3 | 1*-2 | 2-3 | 3-4 |
| V8 | 87 | 65 | 3-4 | 1 | 2-3 | 3-4 |
| 9 | 78 | 60 | 2-3 | 1 | 2 | 2 |
| 10 | 80 | 60 | 2 | 1 | 2 | 2-3 |
| 11 | 78 | 63 | 2 | 1*-2 | 2 | 2 |
| 12 | 80 | 62 | 2 | 1-2 | 1-2 | 1-2 |
| 13 | 82 | 64 | 2 | 1-2 | 1-2 | 1-2 |
| 14 | 79 | 67 | 2 1-2 | | 1 | 1 |
| 15 | 75 | 55 | 1-2* | 1-2 | 2-3 | 1-2 |
| 16 | 80 | 63 | 2 | 1-2 | 1-2 | 1 |
| 17 | 82 | 60 | 2 | 1-2 | 1 | 1-2 |
| 18 | 76 | 66 | 1-2* | 1-2 | 1 | 1-2 |
| 19 | 78 | 62 | 2-3 | 2 | 2 | 2 |
| 20 | 81 | 61 | 2-3 | 2 | 2 | 1-2 |

| | | | | | | |
|---|---|---|---|---|---|---|
| * Unterstreichung bedeutet der gemessene wert liegt näher an dem unterstrichenen Wert. | | | | | | |

## Patentansprüche

1. Kosmetisches Mittel, enthaltend wenigstens ein wasserlösliches oder wasserdispergierbares Polymer, das
a) wenigstens ein α,β-ethylenisch ungesättigtes Monomer der allgemeinen Formel I 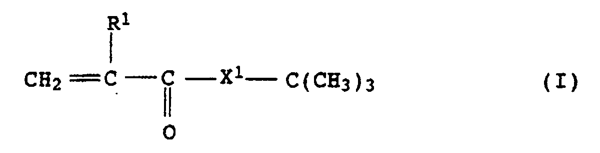 worin
R¹ für Wasserstoff oder C₁-C₈-Alkyl steht, und
X¹ für O oder NR² steht, wobei R² für Wasserstoff, C₁-C₈-Alkyl oder C₅-C₈-Cycloalkyl steht,
b) wenigstens eine α,β-ethylenisch ungesättigte Mono- und/oder Dicarbonsäure,
c) wenigstens eine Verbindung mit mindestens einer α,β-ethylenisch ungesättigten Doppelbindung und mindestens 5 Alkylenoxideinheiten pro Molekül,
d) wenigstens eine Verbindung mit mindestens einer α,β-ethylenisch ungesättigten Doppelbindung und mindestens einem geradkettigen oder verzweigten C₈-C₃₀-Alkyl- oder -Alkylenrest pro Molekül,
einpolymerisiert enthält, wobei die Komponenten c) und/oder d) teilweise oder vollständig durch eine Komponente e) ersetzt sein können, wobei
e) für wenigstens eine Verbindung mit mindestens einer α,β-ethylenisch ungesättigten Doppelbindung, mindestens 5 Alkylenoxideinheiten und mindestens einem geradkettigen oder verzweigten C₈-C₃₀-Alkyl- oder -Alkylenrest pro Molekül steht,
oder die Salze davon.

2. Mittel nach Anspruch 1, wobei die Komponente c) ausgewählt ist unter Polyetheracrylaten der allgemeinen Formel II 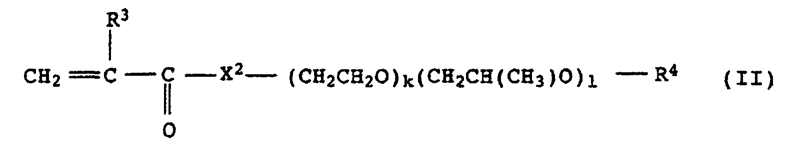 worin
die Reihenfolge der Alkylenoxideinheiten beliebig ist,
k und l unabhängig voneinander für eine ganze Zahl von 0 bis 50 stehen, wobei die Summe aus k und l mindestens 5 beträgt,
R³ für Wasserstoff oder C₁-C₈-Alkyl steht, und
R⁴ für Wasserstoff oder C₁-C₆-Alkyl steht,
K² für O oder NR² steht, wobei R² für Wasserstoff, C₁-C₈-Alkyl oder C₅-C₈-Cycloalkyl steht.

3. Mittel nach einem der Ansprüche 1 oder 2, wobei die Komponente d) ausgewählt ist unter Verbindungen der allgemeinen Formel III 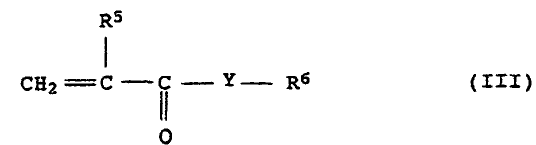 worin
R⁵ für Wasserstoff oder C₁-C₈-Alkyl steht,
R⁶ für einen geradkettigen oder verzweigten C₈-C₃₀-Alkylrest steht, und
Y für O oder NR⁷ steht, wobei R⁷ für Wasserstoff, C₁-C₈-Alkyl oder C₅- bis C₈-Cycloalkyl steht.

4. Mittel nach einem der vorhergehenden Ansprüche, wobei die Komponente e) ausgewählt ist unter
e1) Polyetheracrylaten der allgemeinen Formel II, wie in Anspruch 2 definiert, worin R⁴ für C₈-C₃₀-Alkyl steht,
e2) Alkylenoxidgruppen enthaltenden urethan(meth)acrylaten und Mischungen davon.

5. Mittel nach Anspruch 4, wobei die Komponente e2) die folgenden Verbindungen: f, g und h; oder f, h, i und m; oder g und l; oder i, l und m; oder f, i, l und m; oder f, h, k und m; sowie ggf. weitere Verbindungen eingebaut enthält, wobei
f) für wenigstens ein Diisocyanat steht,
g) für wenigstens eine Verbindung der allgemeinen Formel IV
R⁸-O-(CH₂CH₂O)ₘ(CH₂CH(CH₃)O)ₙ-H (IV)
steht, worin
die Reihenfolge der Alkylenoxideinheiten beliebig ist,
R⁸ für einen geradkettigen oder verzweigten C₈-C₃₀-Alkylrest steht,
m und n unabhängig voneinander für eine ganze Zahl von 0 bis 50 stehen, wobei die Summe aus m und n mindestens 5 beträgt,
h) für wenigstens eine α,β-ethylenisch ungesättigte Verbindung steht, die zusätzlich wenigstens eine gegenüber Isocyanatgruppen reaktive Gruppe pro Molekül enthält,
i) für eine Verbindung steht, die ausgewählt ist unter einwertigen Alkoholen, Diolen, Aminen, Diaminen und Aminoalkoholen mit mindestens einem geradkettigen oder verzweigten C₈-C₃₀-Alkyl- oder -Alkylenrest pro Molekül, und Mischungen davon,
k) für wenigstens ein aliphatisches, cycloaliphatisches oder aromatisches Monoisocyanat steht,
l) für wenigstens eine α,β-ethylenisch ungesättigte Verbindung, die zusätzlich wenigstens eine Isocyanatgruppe pro Molekül enthält, steht,
m) für wenigstens eine Verbindung der allgemeinen Formel V
R⁹-(CH₂CH₂O)ₚ(CH₂CH(CH₃)O)_{q}-R¹⁰ (V)
steht, worin
die Reihenfolge der Alkylenoxideinheiten beliebig ist,
p und q die zuvor für m und n angegebenen Bedeutungen besitzen,
R⁹ für OH oder NHR¹¹ steht, wobei R¹¹ für Wasserstoff, C₁-C₈-Alkyl oder C₅-C₈-Cycloalkyl steht,
R¹⁰ für H, CH₂CH₂NHR¹¹ oder CH₂CH(CH₃)NHR¹¹ steht.

6. Mittel nach Anspruch 5, wobei die Komponente e2) zusätzlich wenigstens eine Komponente, die ausgewählt ist unter
n) Verbindungen mit einem Molekulargewicht im Bereich von 56 bis 300, die zwei aktive Wasserstoffatome pro Molekül enthalten,
o) Polytetrahydrofuranen mit zwei aktiven Wasserstoffatomen pro Molekül
p) Polysiloxanen der allgemeinen Formel VI 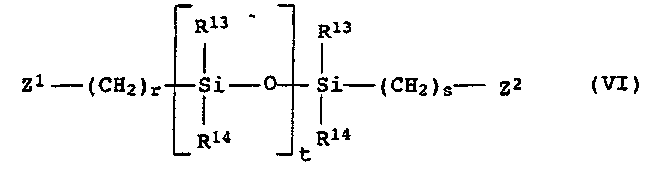
worin
R¹³ und R¹⁴ unabhängig voneinander für C₁-C₄-Alkyl, Benzyl, Phenyl oder einen Rest der Formel VII
-(CH₂)ᵤ-O-(CH₂CH₂O)ᵥ(CH₂CH(CH₃)O)_{w}-H (VII)
stehen, wobei
in der Formel VII die Reihenfolge der Alkylenoxideinheiten beliebig ist,
u für eine ganze Zahl von 1 bis 8 steht,
v und w unabhängig voneinander für eine ganze Zahl von 0 bis 200 stehen, wobei die Summe aus v und w > 0 ist,
z¹ und z² unabhängig voneinander für OH, NHR¹⁵ oder einen Rest der Formel VII stehen, wobei R¹⁵ für Wasserstoff, C₁-C₆-Alkyl oder C₅-C₈-Cycloalkyl steht,
r und s unabhängig voneinander für 2 bis 8 stehen,
t für 3 bis 50 steht,
und Mischungen davon, eingebaut enthält.

7. Mittel nach einem der vorhergehenden Ansprüche, enthaltend ein Polymer, das
- 40 bis 85 Gew.-%, bevorzugt 45 bis 80 Gew.-%, wenigstens einer Komponente a),
- 10 bis 30 Gew.-%, bevorzugt 15 bis 28 Gew.-%, wenigstens einer Komponente b),
- 1 bis 20 Gew.-%, bevorzugt 2 bis 15 Gew.-%, wenigstens einer Komponente c),
- 1 bis 30 Gew.-%, bevorzugt 2 bis 25 Gew.-%, wenigstens einer Komponente d),
einpolymerisiert enthält, wobei die Komponenten c) und/oder d) teilweise oder vollständig durch eine Komponente e) ersetzt sein können.

8. Mittel nach einem der vorhergehenden Ansprüche in Form eines Haarbehandlungsmittels, insbesondere in Form eines Haarsprays.

9. Mittel nach Anspruch 8, enthaltend
a) 0,5 bis 20 Gew.-% mindestens eines in Wasser löslichen oder dispergierbaren Polymers, wie in einem der Ansprüche 1 bis 8 definiert,
b) 30 bis 99,5 Gew.-%, bevorzugt 40 bis 99 Gew.-%, wenigstens eines Lösungsmittels, ausgewählt unter Wasser, wassermischbaren Lösungsmittel und Mischungen davon,
c) 0 bis 70 Gew.-% eines Treibmittels,
d) 0 bis 10 Gew.-% mindestens eines von a) verschiedenen, in Wasser löslichen oder dispergierbaren Haarpolymers,
e) 0 bis 0,3 Gew.-% mindestens eines wasserunlöslichen Silicons,
f) 0 bis 1 Gew.-% mindestens eines nichtionischen, siloxanhaltigen, in Wasser löslichen oder dispergierbaren Polymers.

10. Verwendung der Polymere, wie in einem der Ansprüche 1 bis 7 definiert, als Hilfsmittel in der Kosmetik, insbesondere in der Haarkosmetik, in der Pharmazie, bevorzugt in Beschichtungsmitteln oder Bindemitteln für feste Arzneiformen, sowie in Beschichtungsmitteln für die Textil-, Papier-, Druck-, Leder- und Klebstoffindustrie.

## Claims

1. A cosmetic composition comprising at least one water-soluble or water-dispersible polymer which comprises, in copolymerized form,
a) at least one α,β-ethylenically unsaturated monomer of the formula I 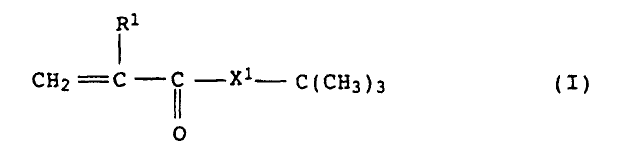 in which
R¹ is hydrogen or C₁-C₈-alkyl, and
X¹ is O or NR², where R² is hydrogen, C₁-C₈-alkyl or C₅-C₈-cycloalkyl,
b) at least one α,β-ethylenically unsaturated mono- and/or dicarboxylic acid,
c) at least one compound having at least one α,β-ethylenically unsaturated double bond and at least 5 alkylene oxide units per molecule,
d) at least one compound having at least one α,β-ethylenically unsaturated double bond and at least one straight-chain or branched C₈-C₃₀-alkyl or alkylene radical per molecule,
where the components c) and/or d) can be partially or completely replaced by a component e), where
e) is at least one compound having at least one α,β-ethylenically unsaturated double bond, at least 5 alkylene oxide units and at least one straight-chain or branched C₈-C₃₀-alkyl or alkylene radical per molecule,
or the salts thereof.

2. A composition as claimed in claim 1, where component c) is chosen from polyether acrylates of the formula II 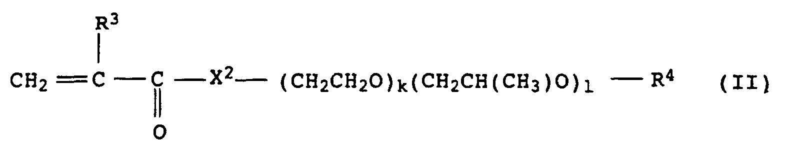 in which
the order of the alkylene oxide units is arbitrary,
k and l independently of one another are an integer from 0 to 50, the sum k + l being at least 5,
R³ is hydrogen or C₁-C₈-alkyl, and
R⁴ is hydrogen or C₁-C₆-alkyl,
X² is O or NR², where R² is hydrogen, C₁-C₈-alkyl or C₅-C₈-cycloalkyl.

3. A composition as claimed in either of claims 1 and 2, where component d) is chosen from compounds of the formula III 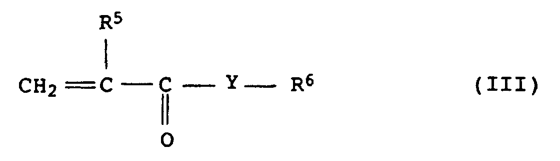 in which
R⁵ is hydrogen or C₁-C₈-alkyl,
R⁶ is a straight-chain or branched C₈-C₃₀-alkyl radical, and
Y is O or NR⁷, where R⁷ is hydrogen, C₁-C₈-alkyl or C₅- to C₈-cycloalkyl.

4. A composition as claimed in one of the preceding claims, where component e) is chosen from
e1) polyether acrylates of the formula II, as defined in claim 2, in which R⁴ is C₈-C₃₀-alkyl,
e2) urethane (meth)acrylates containing alkylene oxide groups and mixtures thereof.

5. A composition as claimed in claim 4, where component e2) comprises, in incorporated form, the following compounds: f, g and h; or f, h, i and m; or g and l; or i, l and m; or f, i, l and m; or f, h, k and m; and optionally other compounds, where
f) is at least one diisocyanate,
g) is at least one compound of the formula IV
R⁸-O-(CH₂CH₂O)ₘ(CH₂CH(CH₃)O)ₙ-H (IV)
in which
the order of the alkylene oxide units is arbitrary,
R⁸ is a straight-chain or branched C₈-C₃₀-alkyl radical,
m and n independently of one another are an integer from 0 to 50, the sum m + n being at least 5,
h) is at least one α,β-ethylenically unsaturated compound which, per molecule, additionally contains at least one group which is reactive toward isocyanate groups,
i) is a compound chosen from monohydric alcohols, diols, amines, diamines and aminoalcohols having at least one straight-chain or branched C₈-C₃₀-alkyl or -alkylene radical per molecule, and mixtures thereof,
k) is at least one aliphatic, cycloaliphatic or aromatic monoisocyanate,
l) is at least one α,β-ethylenically unsaturated compound which additionally contains at least one isocyanate group per molecule,
m) is at least one compound of the formula V
R⁹-(CH₂CH₂O)ₚ(CH₂CH(CH₃)O)_{q}-R¹⁰ (V)
in which
the order of the alkylene oxide units is arbitrary,
p and q are as defined above for m and n,
R⁹ is OH or NHR¹¹, where R¹¹ is hydrogen, C₁-C₈-alkyl or C₅-C₈-cycloalkyl,
R¹⁰ is H, CH₂CH₂NHR¹¹ or CH₂CH(CH₃)NHR¹¹.

6. A composition as claimed in claim 5, where component e2) additionally comprises, in incorporated form, at least one component chosen from
n) compounds having a molecular weight in the range from 56 to 300 which contain two active hydrogen atoms per molecule,
o) polytetrahydrofurans having two active hydrogen atoms per molecule
p) polysiloxanes of the formula VI 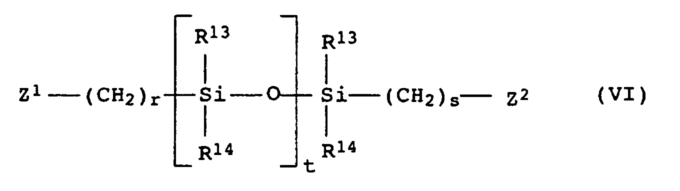 in which
R¹³ and R¹⁴ independently of one another are C₁-C₄-alkyl, benzyl, phenyl or a radical of the formula VII
-(CH₂)ᵤ-O-(CH₂CH₂O)ᵥ(CH₂CH(CH₃)O)_{w}-H (VII)
where
in formula VII the order of the alkylene oxide units is arbitrary,
u is an integer from 1 to 8,
v and w independently of one another are an integer from 0 to 200, the sum v + w being > 0,
Z¹ and Z² independently of one another are OH, NHR¹⁵ or a radical of the formula VII, where R¹⁵ is hydrogen, C₁-C₆-alkyl or C₅-C₈-cycloalkyl,
r and s independently of one another are from 2 to 8,
t is from 3 to 50,
and mixtures thereof.

7. A composition as claimed in one of the preceding claims, comprising a polymer which comprises, in copolymerized form,
- from 40 to 85% by weight, preferably from 45 to 80% by weight, of at least one component a),
- from 10 to 30% by weight, preferably from 15 to 28% by weight, of at least one component b),
- from 1 to 20% by weight, preferably from 2 to 15% by weight, of at least one component c),
- from 1 to 30% by weight, preferably from 2 to 25% by weight, of at least one component d),
where components c) and/or d) can be partially or completely replaced by a component e).

8. A composition as claimed in one of the preceding claims in the form of a hair-treatment composition, in particular in the form of a hair spray.

9. A composition as claimed in claim 8, comprising
a) from 0.5 to 20% by weight of at least one water-soluble or -dispersible polymer, as defined in one of claims 1 to 9,
b) from 30 to 99.5% by weight, preferably from 40 to 99% by weight, of at least one solvent chosen from water, water-miscible solvents and mixtures thereof,
c) from 0 to 70% by weight of a propellant,
d) from 0 to 10% by weight of at least one water-soluble or -dispersible hair polymer which is different from a),
e) from 0 to 0.3% by weight of at least one water-insoluble silicone,
f) from 0 to 1% by weight of at least one nonionic, siloxane-containing, water-soluble or -dispersible polymer.

10. The use of the polymers as defined in one of claims 1 to 7 as auxiliaries in cosmetics, in particular in hair cosmetics, in pharmaceuticals, preferably in coating compositions or binders for solid medicament forms, and in coating compositions for the textile, paper, printing, leather and adhesive industries.

## Revendications

1. Composition cosmétique, contenant au moins un polymère soluble dans l'eau ou dispersible à l'eau, qui
a) contient au moins un monomère α,β-éthyléniquement insaturé de la formule générale I 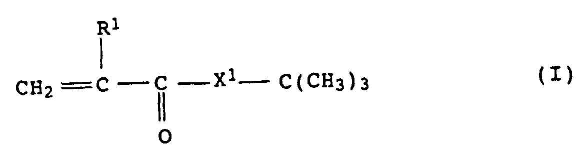 dans laquelle
R¹ représente de l'hydrogène ou un radical alkyle en C₁ à C₈, et
X¹ représente 0 ou NR², où R² représente de l'hydrogène ou un radical alkyle en C₁ à C₈ ou cycloalkyle en C₅ à C₈,
b) au moins un acide mono- et/ou dicarboxylique α,β-éthyléniquement insaturé,
c) au moins un composé ayant au moins une double liaison α,β-éthyléniquement insaturée et au moins 5 unités d'oxyde d'alkylène par molécule,
d) au moins un composé ayant au moins une double liaison α,β-éthyléniquement insaturée et au moins un reste alkyle ou alkylène en C₈ à C₃₀ à chaîne linéaire ou ramifiée par molécule,
à l'état copolymérisé, où les composants c) et/ou d) peuvent être remplacés partiellement ou totalement par un composant e), auquel cas
e) représente au moins un composé ayant au moins une double liaison α,β-éthyléniquement insaturée et au moins 5 unités d'oxyde d'alkylène et au moins un reste alkyle ou alkylène en C₈ à C₃₀ à chaîne linéaire ou ramifiée par molécule, ou leurs sels.

2. Composition selon la revendication 1, où le composant c) est choisi parmi des polyéther acrylates de la formule générale II 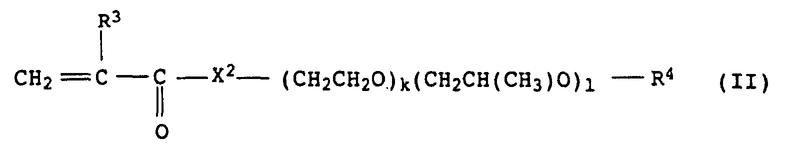 dans laquelle
la succession des unités d'oxyde d'alkylène est quelconque,
k et l représentent indépendamment l'un de l'autre un nombre entier de 0 à 50, la somme de k et l étant d'au moins 5,
R³ représente de l'hydrogène ou un radical alkyle en C₁ à C₈, et
R⁴ représente de l'hydrogène ou un radical alkyle en C₁ à C₆, et
X² représente O ou NR², où R² représente de l'hydrogène ou un radical alkyle en C₁ à C₈ ou cycloalkyle en C₅ à C₈.

3. Composition selon l'une des revendications 1 ou 2, où le composant d) est choisi parmi des composés de la formule générale III 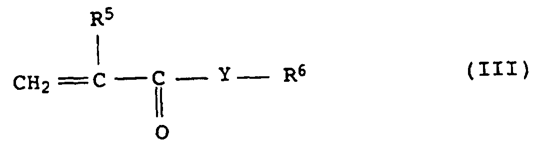 dans laquelle
R⁵ représente de l'hydrogène ou un radical alkyle en C₁ à C₈,
R⁶ représente un reste alkyle ou alkylène en C₈ à C₃₀ à chaîne linéaire ou ramifiée, et
Y représente O ou NR⁷, où R⁷ représente de l'hydrogène ou un radical alkyle en C₁ à C₈ ou cycloalkyle en C₅ à C₈.

4. Composition selon l'une des revendications qui précèdent, où le composant e) est choisi parmi
e1) des polyéther acrylates de la formule générale II, comme définis dans la revendication 2, où R⁴ représente un radical alkyle en C₈ à C₃₀,
e2) des (méth)acrylates d'uréthanne contenant des groupes d'oxyde d'alkylène,
et leurs mélanges.

5. Composition selon la revendication 4, où le composant e2) contient les composés suivants: f, g et h, ou f, h, i et m, ou g et l, ou i, l et m, ou f, i, l et m, ou f, h, k et m, ainsi qu'éventuellement d'autres composés incorporés, avec
f) étant au moins un diisocyanate,
g) étant au moins un composé de la formule générale IV
R⁸-O-(CH₂CH₂O)ₘ(CH₂CH(CH₃)O)ₙ-H (IV)
dans laquelle
la succession des unités d'oxyde d'alkylène est quelconque,
R⁸ représente un radical alkylène en C₈ à C₃₀ à chaîne linéaire ou ramifiée,
m et n représentent indépendamment l'un de l'autre un nombre entier de 0 à 50, la somme de k et l étant d'au moins 5,
h) étant au moins un composé α,β-éthyléniquement insaturé, qui contient en outre au moins un groupe réactif vis-à-vis des isocyanates par molécule,
i) étant un composé qui est choisi parmi des alcools monovalents, des diols, des amines, des diamines et des aminoalcools avec au moins un reste alkyle ou alkylène en C₈ à C₃₀ à chaîne linéaire ou ramifiée par molécule, et leurs mélanges,
k) étant au moins un monoisocyanate aliphatique, cycloaliphatique ou aromatique,
l) étant au moins un composé α,β-éthyléniquement insaturé, qui contient en outre au moins un groupe isocyanate par molécule,
m) étant au moins un composé de la formule générale V
R⁹-(CH₂CH₂O)ₚ(CH₂CH(CH₃)O)_{q}-R¹⁰ (V)
dans laquelle
la succession des unités d'oxyde d'alkylène est quelconque,
p et q ont les significations indiquées plus haut pour m et n,
R⁹ représente OH ou NR¹¹, où R¹¹ est de l'hydrogène ou un radical alkyle en C₁ à C₈ ou cycloalkyle en C₅ à C₈,
R¹⁰ représente H, CH₂CH₂NHR¹¹ ou CH₂CH(CH₃) NHR¹¹.

6. Composition selon la revendication 5, où le composant e2) contient en outre incorporé au moins un composant choisi parmi
n) des composés d'un poids moléculaire de l'ordre de 56 à 300, qui contiennent deux atomes d'hydrogène actif par molécule,
o) des polytétrahydrofurannes contenant deux atomes d'hydrogène actif par molécule,
p) des polysiloxanes de la formule générale VI 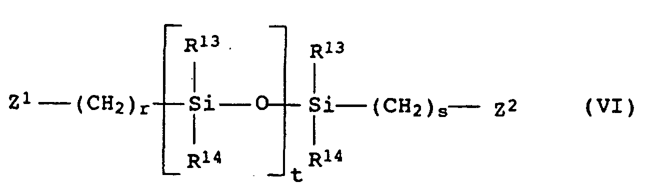
dans laquelle
R¹³ et R¹⁴ représentent indépendamment l'un de l'autre un radical alkyle en C₁ à C₄, benzyle, phényle ou un reste de la formule VII
-(CH₂)ᵤ-O-(CH₂CH₂O)ᵥ(CH₂CH(CH₃)O)_{w}-H (VII)
où
dans la formule VII, la succession des unités d'oxyde d'alkylène est quelconque,
u représente un nombre entier de 1 à 8,
v et w représentent indépendamment l'un de l'autre un nombre entier de 0 à 200, la somme de v et w étant >0,
Z¹ et Z² représentent indépendamment l'un de l'autre OH, NHR¹⁵ ou un reste de la formule VII, où R¹⁵ représente de l'hydrogène ou un radical alkyle en C₁ à C₆ ou cycloalkyle en C₅ à C₈,
r et s ont indépendamment l'un de l'autre une valeur de 2 à 8,
t a une valeur de 3 à 50,
et leurs mélanges.

7. Composition selon l'une quelconque des revendications qui précèdent, contenant un polymère, qui contient à l'état copolymérisé
- de 40 à 85% en poids, de préférence de 45 à 80% en poids, d'au moins un composant a),
- de 10 à 30% en poids, de préférence de 15 à 28% en poids d'au moins un composant b),
- de 1 à 20% en poids, de préférence de 2 à 15% en poids d'au moins un composant c),
- de 1 à 30% en poids, de préférence de 2 à 25% en poids d'au moins un composant d),
les composants c) et/ou d) pouvant être partiellement ou totalement remplacés par un composant e).

8. Composition selon l'une quelconque des revendications qui précèdent, sous la forme d'un produit de soin capillaire, en particulier sous la forme d'une laque pour cheveux.

9. Composition selon la revendication 8, contenant
a) de 0,5 à 20% en poids d'au moins un polymère soluble dans l'eau ou dispersible à l'eau, comme défini dans l'une quelconque des revendications 1 à 8,
b) de 30 à 99,5% en poids, de préférence de 40 à 99% en poids, d'au moins un solvant, choisi parmi l'eau, des solvants miscibles à l'eau et leurs mélanges,
c) de 0 à 70% en poids d'un agent propulseur,
d) de 0 à 10% en poids d'au moins un polymère capillaire soluble dans l'eau ou dispersible à l'eau, différent de a),
e) de 0 à 0,3% en poids d'au moins un silicone insoluble dans l'eau,
f) de 0 à 1% en poids d'au moins un polymère non ionique contenant du siloxane, soluble dans l'eau ou dispersible à l'eau.

10. Utilisation des polymères tels que définis dans l'une quelconque des revendications 1 à 7 comme adjuvants en cosmétique, en particulier dans la cosmétique capillaire, en pharmacie, de préférence dans des agents d'enrobage ou des liants pour formes de médicaments solides, ainsi que dans des agents de revêtement pour l'industrie des textiles, du papier, de l'imprimerie, du cuir et des adhésifs.
